(19) Europäisches Patentamt
European Patent Office
Office européen des brevets

(11) Numéro de publication : **0 319 412 B1**

# (12) FASCICULE DE BREVET EUROPEEN

(45) Date de publication du fascicule du brevet : **11.03.92 Bulletin 92/11**

(51) Int. Cl.[5] : **C07D 311/30,** C07D 311/32, C07H 17/07, A61K 31/35

(21) Numéro de dépôt : **88403024.8**

(22) Date de dépôt : **01.12.88**

(54) **Nouveaux dérivés flavonoides pipérazinyl-2 oxo-2 éthylène substitués, leurs procédés de préparation et les compositions pharmaceutiques qui les contiennent.**

(30) Priorité : **01.12.87 FR 8716619**

(43) Date de publication de la demande : **07.06.89 Bulletin 89/23**

(45) Mention de la délivrance du brevet : **11.03.92 Bulletin 92/11**

(84) Etats contractants désignés : **AT BE CH DE ES FR GB GR IT LI LU NL SE**

(56) Documents cités :
**US-A- 3 002 979**
**US-A- 3 810 896**
**US-A- 3 976 790**

(73) Titulaire : **ADIR ET COMPAGNIE**
**1 rue Carle Hébert**
**F-92415 Courbevoie Cédex (FR)**

(72) Inventeur : **Rolland, Yves**
**76 Rue Jean-Jaurès**
**F-92170 Vanves (FR)**
Inventeur : **Duhault, Jacques**
**14 bis Rue Paul Demange**
**F-78290 Croissy sur Seine (FR)**

EP 0 319 412 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne de nouveaux dérivés flavonoïdes pipérazinyl-2 oxo-2 éthylène substitués, leurs procédés de préparation et les compositions pharmaceutiques qui les contiennent.

Il est déjà connu que les flavonoïdes et certains de leurs dérivés produits d'hémisynthèse possèdent des activités physiologiques et thérapeutiques importantes : en particulier, ils réduisent la fragilité et/ou la perméabilité capillaire et ont une action anti-inflammatoire. (Burger's Medicinal Chemistry, 4th Ed., Part III, p. 1258-1259 Manfred Wolff edt., J. Wiley, N.Y.) (Demandes de brevet françaises N°7.329.235 et N°2.183.612 ; Brevet USA N°3.810.896).

D'autres dérivés doués de propriétés coronaro-dilatatrices sont aussi décrits (Brevet US N°3.002.979). Enfin, quelques composés d'hémisynthèse d'hespérétine sont connus et trouvent leurs applications en bromatologie (Brevet US N°3.976.790).

La demanderesse a maintenant découvert que certains dérivés flavonoïdes pipérazinyl-2 oxo-2 éthylène substitués, de structure originale, sont doués de propriétés pharmacologiques très intéressantes. En effet, les composés de la présente invention agissent sur les médiateurs biochimiques impliqués dans les troubles dus à l'insuffisance veineuse. En plus, lors des essais in vivo, les produits de l'invention ont montré un effet inhibiteur très important vis-à-vis de la constitution de l'oedème.

La présente invention a plus particulièrement pour objet les dérivés flavonoïdes pipérazinyl-2 oxo-2 éthylène substitués de formule générale I:

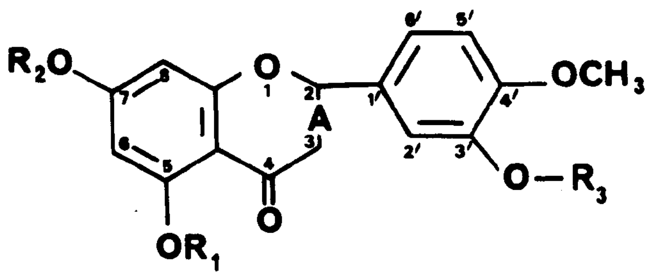

(I)

dans laquelle :

– A représente une simple ou une double liaison,

– $R_1$, $R_3$ identiques ou différents représentent chacun un atome d'hydrogène, un radical alcoxy carbonyl méthylène de formule $-CH_2CO_2R_4$ (dans laquelle $R_4$ représente un radical alkyle de 1 à 5 atomes de carbone) ou un radical de formule W :

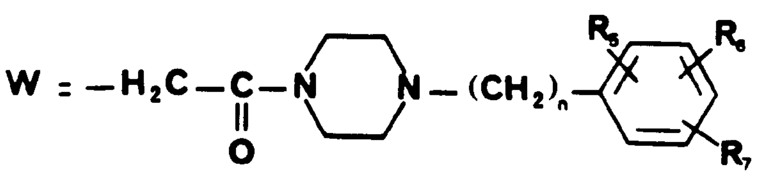

(dans laquelle n est égal à 0 ou 1 et $R_5$, $R_6$, $R_7$ identiques ou différents représentent chacun un atome d'hydrogène ou d'halogène, un radical hydroxyle, un radical trifluorométhyle, un radical alkyle inférieur renfermant de 1 à 5 atomes de carbone ou un radical alcoxy renfermant de 1 à 5 atomes de carbone),

– $R_2$ représente un atome d'hydrogène, un radical alcoxy carbonyl méthylène de formule $-CH_2CO_2R_4$, un radical de formule W, ou une molécule de β-glucose ou rutinose liée à l'oxygène auquel elle est attachée avec une liaison osidique,

à condition toutefois qu'au moins ou $R_1$, ou $R_2$, ou $R_3$ représente toujours un radical de formule W,

et leurs sels d'addition à un acide minéral ou organique pharmaceutiquement acceptable.

La présente invention a également pour objet le procédé de préparation de composés de formule générale I, caractérisé en ce que :

**soit :**

l'on fait réagir la diosmine, composé de formule II :

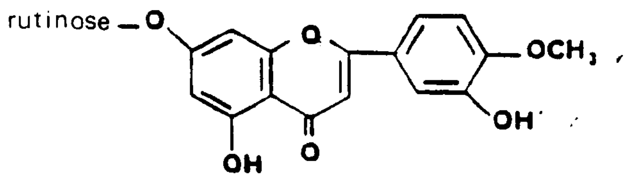

(II)

avec un chlorure d'alkyle de formule III renfermant les composés de formules générales IIIa et IIIb :

$ClCH_2CO_2R_4$ IIIa

Cl W IIIb

(III)

dans lesquels $R_4$ et W ont les mêmes significations que pour la formule I, dans un solvant organique azoté polaire, en présence d'un sel minéral acide de métal alcalin à une température comprise entre 80°-120°C, pour obtenir

- **ou**

avec les composés de formule IIIa, les composés de formule générale IV :

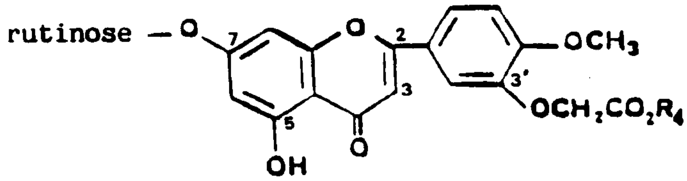

(IV)

dans laquelle $R_4$ a la signification indiquée ci-dessus pour la formule I,

que l'on soumet à une hydrolyse acide en présence d'un acide minéral concentré et à une température comprise entre 35°-55°C pour former le composé de formule V :

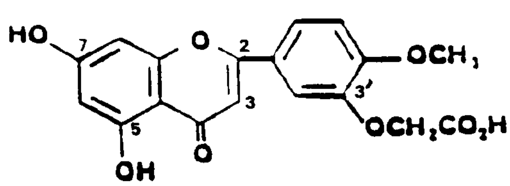

(V)

lequel ensuite :

- soit :

que l'on soumet à une acétylation à l'aide de l'anhydride acétique dans un solvant organique azoté basique et à une température ambiante pour former le composé de formule VI :

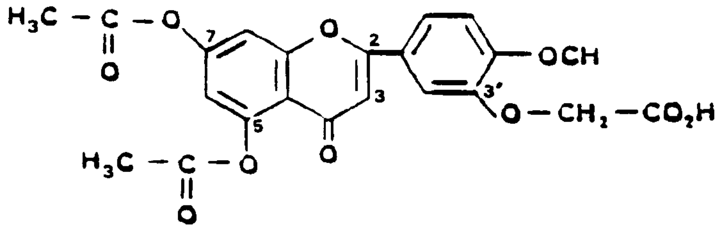

(VI)

que l'on fait réagir avec un dérivé de la pipérazine de formule générale VII :

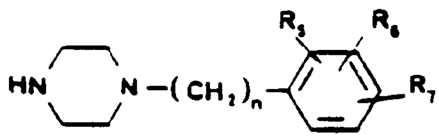

(VII)

dans laquelle n, $R_5$, $R_5$, $R_7$ ont la même signification que pour la formule I, en présence d'une amine tertiaire de petit poids moléculaire et de chloroformiate d'éthyle, à une température comprise entre 0°-20°C pour former les composés de formule générale VIII :

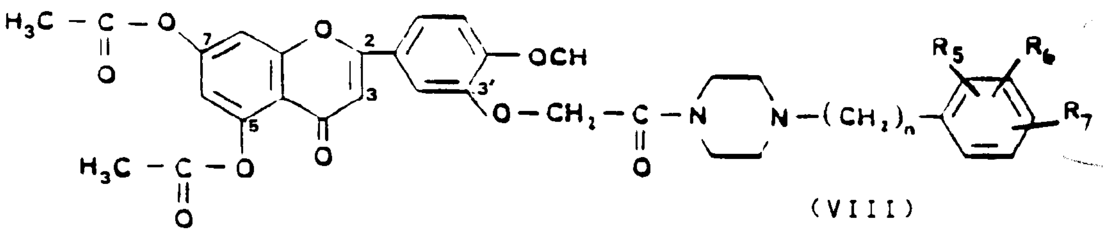

(VIII)

dans laquelle n, $R_5$, $R_6$, $R_7$ ont la signification indiquée ci-dessus,

que l'on soumet ensuite à une désacétylation en solution dans le diméthylformamide en présence d'un sel minéral acide d'un métal alcalin à une température comprise entre 80°-120°C, pour former les composés de formule générale $I_A$ :

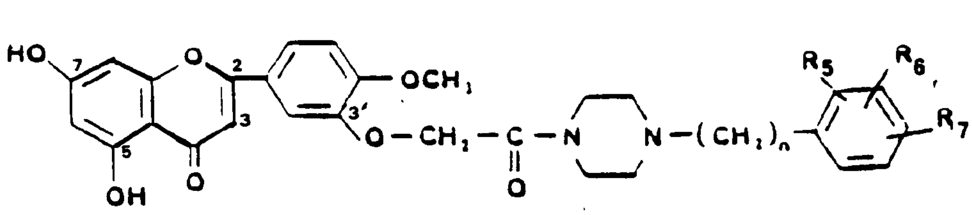

($I_A$)

dans laquelle la signification de n, $R_5$, $R_6$, $R_7$ reste identique à celle donnée précédemment,

que l'on condense avec un composé de formule générale III pour former des composés de formule générale $I_B$ :

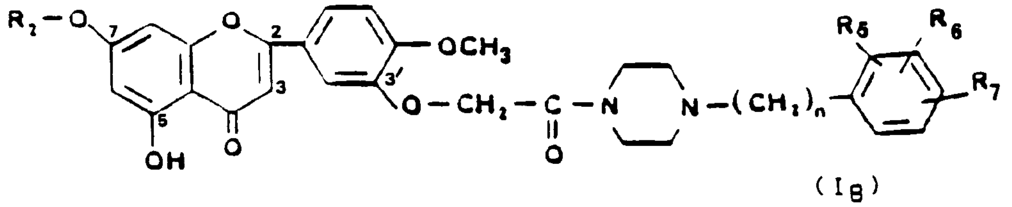

(IB)

(IB)

dans laquelle n, $R_5$, $R_6$ et $R_7$ ont la signification indiquée ci-dessus et $R_2$ représente un radical alcoxy carbonyl méthylène de formule $-CH_2CO_2R_4$ ou un radical de formule W,

- soit :

que l'on estérifie avec un alcool primaire ou secondaire contenant de 1 à 5 atomes de carbone, en milieu anhydre à une température comprise entre 80°-110°C et en présence de l'acide tosylique pour former les composés de formule générale IX :

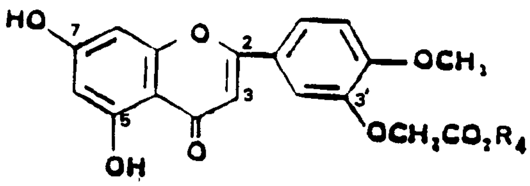

(IX)

dans laquelle $R_4$ a la même signification que pour la formule IV,

que l'on fait réagir

■ ou avec un chlorure d'alkyle de formule générale $III_B$ pour former les composés de formule générale $I_C$ :

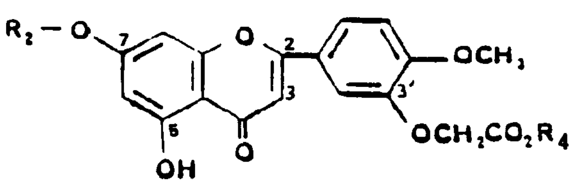

($I_C$)

dans laquelle $R_2$ représente un radical de formule W,

■ ou avec un chlorure d'alkyle de formule $III_A$ pour former les composés de formule générale X :

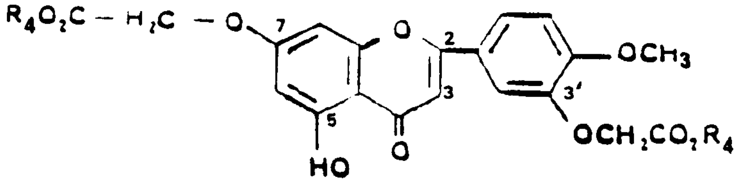

(X)

dans laquelle $R_4$ a la signification indiquée ci-dessus pour la formule I,

que l'on condense avec un composé de formule générale $III_B$ pour obtenir les composés de formule générale $I_D$ :

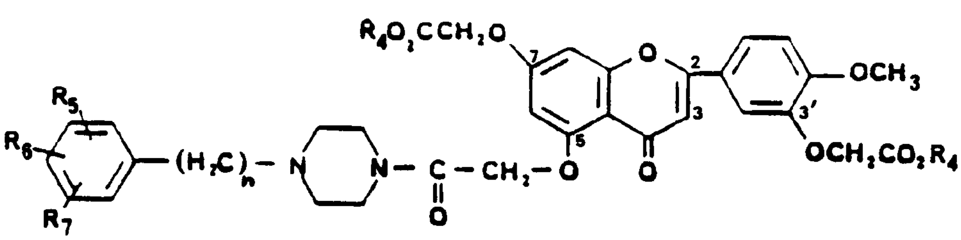

($I_D$)

dans laquelle la signification de n, $R_4$, $R_5$, $R_6$ et $R_7$ reste identique à celle donnée ci-dessus,

- <u>ou</u>

avec les composés de formule $III_B$, les composés de formule générale $I_E$,

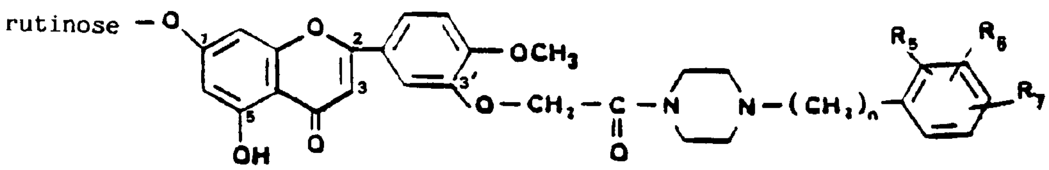

$(I_E)$

dans laquelle n, $R_5$, $R_6$, $R_7$ ont la signification indiquée pour la formule I,

que l'on soumet ensuite à une hydrolyse enzymatique à l'aide de naringinase pour former les composés de formule générale $I_F$ :

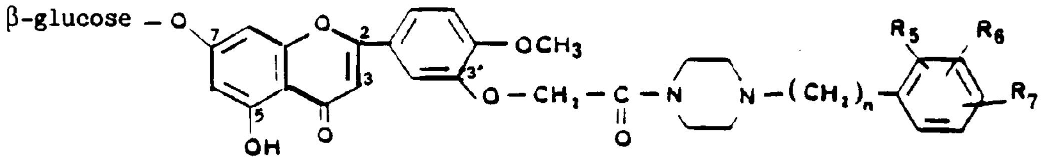

$(I_F)$

$(I_F)$

dans laquelle n, $R_5$, $R_6$ et $R_7$ ont la signification indiquée ci-dessus pour la formule I,

lesquels ensuite,

que l'on soumet à une hydrolyse enzymatique à l'aide de la β-glucosidase pour former les composés de formule générale $I_A$,

que l'on condense ensuite avec un composé de formule générale III pour former les composés de formule générale $I_B$,

**soit :**

l'on fait réagir un composé de formule générale XI :

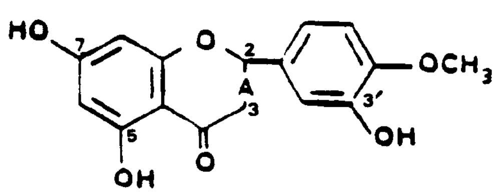

(XI)

dans laquelle A a la même signification que pour la formule I avec une quantité adéquate des composés de formule générale $III_B$,

pour former les composés de formule générale $I_G$ :

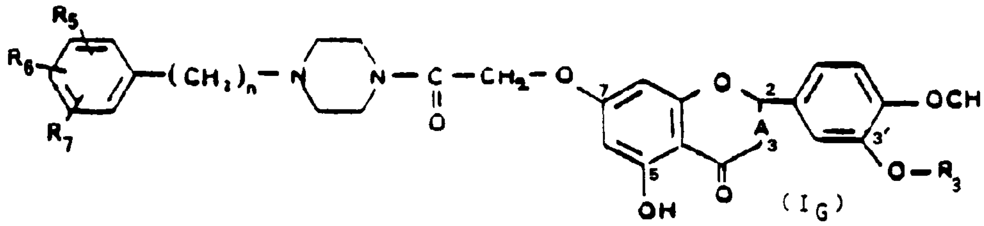

$(I_G)$

dans laquelle A, n, $R_5$, $R_6$ et $R_7$ ont la signification indiquée dans la formule générale I et $R_3$ représente un atome d'hydrogène quand la quantité des composés de formule générale $III_B$ utilisée est environ équimolaire, ou un radical de formule W quand l'alkylation est effectuée avec une quantité de chlorure d'alkyle de formule générale $III_B$ au moins double,

et que l'on salifie ensuite, si l'on désire, les composés des formules $I_A$ à $I_G$, qui forment l'ensemble des composés de formule I, avec un acide organique ou minéral pharmaceutiquement acceptable.

Le procédé de préparation des composés $I_A$ à $I_F$ est illustré dans les planches des formules N°1 à N°3.

Parmi les acides pharmaceutiquement acceptables pour la préparation des sels d'addition aux composés de formule générale I, on peut citer les acides chlorhydrique, phosphorique, fumarique, citrique, oxalique, sulfurique, ascorbique, tartrique, maléïque, mandélique, méthanesulfonique etc...

Les composés de formule générale $III_B$ peuvent être préparés par amidification des dérivés de la pipérazine de formule générale VII à l'aide de chlorure de chloroacétyle. Cette réaction est réalisée dans un solvant organique chloré tel que le dichlorométhane à une température comprise entre 0°-20°C.

Les composés de la présente invention possèdent des propriétés pharmacologiques et biologiques de grand intérêt. De plus, leurs sels d'addition sont très solubles dans l'eau. Ils peuvent donc être avantageusement employés pour la préparation d'injectables.

Les différents tests effectués in vivo ont démontré que les composés de l'invention possèdent une activité antioedémateuse très puissante. Des essais in vitro ont aussi relevé que les composés de l'invention présentent des effets protecteurs, fort intéressants, vis-à-vis de la réaction inflammatoire.

Il est connu que les métabolites de l'oxygène sont impliqués dans les réactions d'inflammation tissulaire tant au niveau de la réaction vasculaire qu'à celui des atteintes tissulaires et cellulaires. (Bruce et col. Lab.Invest., 1982,47,(5),p.412-426). Des essais pharmacologiques réalisés avec les composés de l'invention ont prouvé que ces derniers inhibent la génération de radicaux libres oxygénés dans le milieu extracellulaire. Les résultats des études pharmacologiques ont aussi mis en évidence que les composés de l'invention possèdent une activité inhibitrice sur la dégradation de l'AMP cyclique et comme d'autres inhibiteurs de la phosphodiestérase (Lehmeyer J.E.Jonhston, R.B.Clin.Immunol. Immunopathol., 1978,9,p.482-490) induisent une diminution de la libération de médiateurs de la réaction inflammatoire par les polymorphonucléaires.

L'oedème et l'inflammation tissulaire sont des manifestations de l'insuffisance veineuse. (Arch.Int.Pharmacodyn.Ther.,(1981),254,p.168 ; et Phlebology 85, Nergus D. et Jantet G., Ed. 1981, Libbey J., Lon.Par.)

Les composés de la présente invention trouvent donc leur application dans le traitement de différentes affections vasculaires et en particulier pour l'insuffisance veineuse chronique des membres inférieurs, fonctionnelle et organique, de l'oedème des membres inférieurs, la maladie hémorroïdaire etc...

Les composés de l'invention sont des substances très peu toxiques. En effet, la $DL_{50}$ observée chez le rat et la souris après administration orale est supérieure à 1600 $mg.kg^{-1}$.

L'invention s'étend aussi aux compositions pharmaceutiques renfermant comme principe actif au moins un composé de formule générale I, ou l'un de ses sels avec un acide minéral ou organique pharmaceutiquement compatible, en association avec un ou plusieurs excipients inertes, et appropriés.

Les compositions pharmaceutiques ainsi obtenues sont présentées avantageusement sous des formes diverses telles que par exemple comprimés, dragées, gélules, pommades ou autres préparations galéniques appropriées pour une administration locale, suppositoires, solutions injectables ou buvables.

La posologie peut varier largement en fonction de l'âge, du poids du patient, de la nature et de la sévérité de l'affection ainsi que de la voie d'administration. D'une manière générale, la posologie unitaire s'echelonnera entre 50 et 500 mg et la posologie journalière, utilisable en thérapeutique humaine, entre 50 mg et 1 g.

La voie d'administration préférée est la voie orale ou parentérale.

Les exemples suivants, donnés à titre non-limitatif, illustrent l'invention.

Les points de fusion ont été mesurés selon la technique Micro-Köfler.

**EXEMPLE 1**

**[{[(Triméthoxy-2,3,4 phényl) méthyl]-4 pipérazinyl-1}-2 oxo-2 éthoxy]-7 hydroxy-5 méthoxy-4′ (éthoxy-2 oxo-2 éthoxy)-3′ flavone**

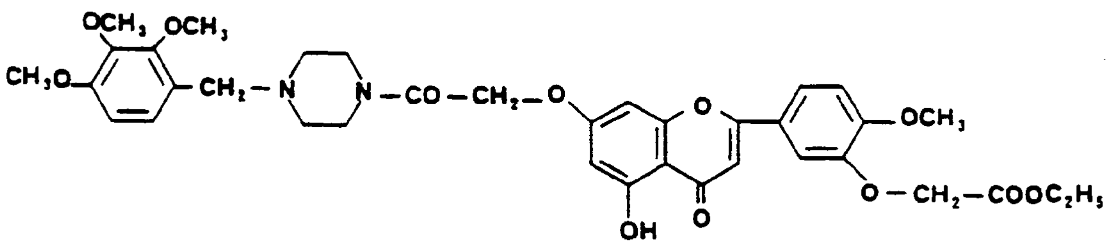

STADE A

O-(éthoxy-2 oxo-2 éthyl)-3′ diosmine

A 50 mmole de diosmine et 52,5 mmole de carbonate acide de potassium, on ajoute 250 ml de diméthylformamide anhydre, puis 215 mmole de chloroacétate d'éthyle, sous azote et agitation au bain d'huile à 110°C. Le mélange réactionnel fortement hétérogène devient plus limpide avec le temps ; la réaction est poursuivie pendant 3 heures et demie à 110°C. Après refroidissement, le milieu réactionnel est filtré sur verre fritté et évaporé à sec au rotavapor et à la pompe à palettes, pour obtenir un résidu d'aspect gélifié.

Rendement : 100%

STADE B

O-carboxyméthyl-3′ diosmétine

Le résidu de l'étape précédente est mis en solution dans 300 ml d'acide chlorhydrique concentré (11 N) et est agité au bain d'huile à 50°C.

Le milieu réactionnel très foncé commence à précipiter. La cristallisation est poursuivie à température ambiante, puis le mélange est filtré sur verre fritté.

Le précipité est lavé à plusieurs reprises à l'eau jusqu'à neutralité des eaux de lavage, séché sous vide, broyé au mortier, puis séché à 50°C à la pompe à palettes.

Rendement : 78%

STADE C

O-éthoxycarbonyl méthyl-3′ diosmétine

L'acide obtenu au stade précédent (14,1 g) et 0,7 g d'acide tosylique sont mis en mélange dans 200 ml d'éthanol absolu; le milieu est porté à reflux sous azote et agitation pendant 5 heures à 100°C. Ensuite, on ajoute 100 ml d'éthanol et on laisse reposer une nuit à température ambiante.

Rendement : 89%

(chloro-1 acétyl)-1 [(triméthoxy-2,3,4 phényl) méthyl]-4 pipérazine

A une solution de 115 mmole de [(triméthoxy-2,3,4 phényl) méthyl]-4 pipérazine dans 200 ml de dichlorométhane, refroidie au bain de glace, on ajoute goutte à goutte en 15 minutes une solution de chlorure de chloroacétyle (au total 115 mmole) dans le dichlorométhane. L'agitation est poursuivie encore 15 minutes, puis le milieu réactionnel est noyé à l'eau glacée, alcalinisé à pH 8 par le carbonate acide de sodium et extrait par le dichlorométhane. La phase organique est lavée à l'eau, séchée sur sulfate de sodium anhydre et évaporée à sec au rotavapor puis à la pompe à palettes.

Rendement : 100%

STADE D

A 20 mmole de O-éthoxycarbonyl méthyl-3′ diosmétine et 30 mmole de carbonate acide de potassium, on ajoute 50 ml de diméthylformamide anhydre sous azote et agitation à 110°C. Après dissolution du composé organique, on additionne 24 mmole de (chloro-1 acétyl)-1 [(triméthoxy-2,3,4 phényl) méthyl]-4 pipérazine. La réaction est poursuivie pendant 2 heures.

Après évaporation du solvant réactionnel sous pression réduite, le résidu obtenu est solubilisé dans environ 75 ml d'acétone et la solution est laissée à cristalliser. Les cristaux formés sont isolés par filtration et ensuite purifiés sur colonne de silice (Merck 9385 ®) en utilisant comme solvant d'élution un mélange de dichlorométhane et d'acétone (4:1 V/V) pour obtenir la [{[(triméthoxy-2,3,4 phényl) méthyl]-4 pipérazinyl-1}-2 oxo-2 éthoxy]-7 hydroxy-5 méthoxy-4′ (éthoxy-2 oxo-2 éthoxy)-3′ flavone pure.

Rendement : 59%

Point de fusion : 103-104°C

Analyse élémentaire :

| | C | H | N |
|---|---|---|---|
| % trouvé | 62,02 | 5,88 | 4,24 |
| % calculé | 62,42 | 5,82 | 4,04 |

Spectre de masse :

Le spectre de masse a été réalisé en désorption ionisation chimique ($NH_3$) ; Température de la source 175°C ; Chauffage du filament 50 à 650 mA à 7 mA/s.
693 m/z [M+H]+ (100%) ; 607 m/z ;
525 m/z ; 511 m/z ; 387 m/z ; 309 m/z ;
267 m/z ; 181 m/z.

Pour préparer le monophosphate de [{[(triméthoxy-2,3,4 phényl) méthyl]-4 pipérazinyl-1}-2 oxo-2 éthoxy]-7 hydroxy-5 méthoxy-4′ (éthoxy-2 oxo-2 éthoxy)-3′ flavone, 1 mmole de la base, en solution dans un mélange de tétrahydrofuranne et de dichlorométhane, a été salifié avec une quantité équimolaire d'acide phosphorique 1N.

Solubilité dans l'eau = 158 g/l.

**EXEMPLE 2**

**[{(triméthoxy-2,3,4 phényl) méthyl]-4 pipérazinyl-1}-2 oxo-2 éthoxy]-5 bis (éthoxy-2 oxo-2 éthoxy)-7,3′ méthoxy-4′ flavone**

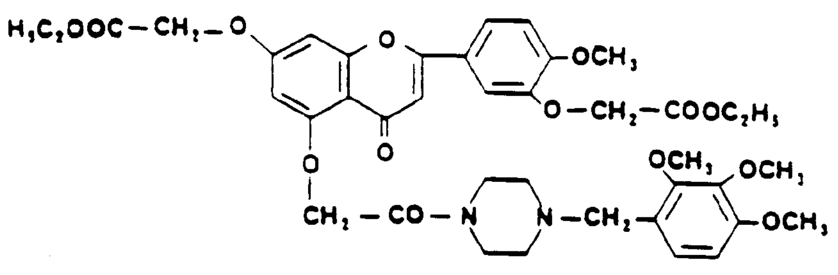

STADE A

Hydroxy-5 bis (éthoxy-2 oxo-2 éthoxy)-7,3′ méthoxy-4′ flavone

A 100 mmole d'O-éthoxycarbonyl méthyl-3′ diosmétine et 120 mmole de carbonate acide de potassium, on ajoute 350 ml de diméthylformamide anhydre sous azote et agitation à 110°C. Après dissolution du composé organique, on additionne 400 mmole de chloroacétate d'éthyle. La réaction est poursuivie pendant 3 heures.

Ensuite, le milieu réactionnel est refroidi et on observe la formation des cristaux. Après 24 heures à température ambiante, le milieu réactionnel est dilué à l'eau et filtré sur verre fritté. Les cristaux sont lavés à l'éthanol et séchés à la pompe à palettes.

Rendement : 90%

STADE B

A 96 mmole du résidu sec obtenu ci-dessus et 480 mmole de carbonate de potassium, on ajoute 350 ml de diméthylformamide anhydre sous azote et agitation à 110°C. Après dissolution du composé organique, on ajoute 105 mmole de (chloro-1 acétyl)-1 [(triméthoxy-2,3,4 phényl) méthyl]-4 pipérazine et on poursuit l'agitation pendant environ 2 heures et demie. Après refroidissement, le milieu réactionnel est filtré sur verre fritté et évaporé à sec. Le résidu est successivement purifié par recristallisation dans l'éthanol, sur colonne de silice (Merck 9385 ®) en utilisant comme solvant d'élution un mélange de dichlorométhane et de méthanol (96 : 4 V/V) et ensuite par recristallisation dans le méthanol.

Rendement global par rapport à la diosmine : 42%.

Point de fusion : 86-88°C

Analyse élémentaire :

| | C | H | N |
|---|---|---|---|
| % trouvé | 61,24 | 6,05 | 3,82 |
| % calculé | 61,69 | 5,95 | 3,60 |

Spectre de masse :

Le spectre de masse a été réalisé en désorption ionisation chimique ($NH_3$) ; Température de la source 175°C ; Chauffage du filament 50 à 650 mA à 7 m A/s.

779 m/z [M+H]+ (100%) ; 693 m/z ; 613 m/z ; 473 m/z; 309 m/z ; 267 m/z ; 181 m/z

Le chlorhydrate de [{[(triméthoxy-2,3,4 phényl) méthyl]-4 pipérazinyl-1}-2 oxo-2 éthoxy]-5 bis (éthoxy-2 oxo-2 éthoxy)-7-3′ méthoxy-4′ flavone a été préparé en salifiant la base correspondante en solution dans le dichlorométhane par une quantité adéquate d'éthanol chlorhydrique 1N.

Solubilité dans l'eau : 163 g/l.

**EXEMPLE 3**

**Rhamnoglucoside-7 de la [{[(triméthoxy-2,3,4 phényl) méthyl]-4 pipérazinyl-1}-2 oxo-2 éthoxy]-3′ méthoxy-4′ hydroxy-5 flavone**

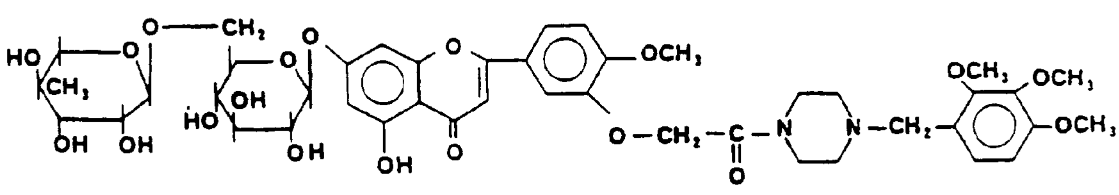

195 mmole de diosmine et 253,5 mmole de carbonate acide de potassium sont mis en suspension dans 800 ml de diméthylformamide anhydre sous azote, agitation et à 100°C. On ajoute ensuite 253,5 mmole de (chloro-1 acétyl)-1 [(triméthoxy-2,3,4 phényl) méthyl]-4 pipérazine en solution dans 175 ml de diméthylformamide et la réaction est poursuivie à cette température pendant 3 heures. Après refroidissement, le milieu réactionnel est filtré sur verre fritté. Le filtrat est dilué à l'eau et extrait par l'acétate d'éthyle quatre fois. L'acétate d'éthyle est eliminé après décantation et ensuite le filtrat est extrait 5 fois par du n-butanol saturé d'eau. Les phases butanoliques bien décantées sont évaporées à sec au rotavapor. Le résidu est repris par un mélange de n-butanol-eau (5:1 V/V). La solution hydroalcoolique ainsi formée est ensuite concentrée et laissée une nuit

à température ambiante. Les cristaux formés sont recristallisés dans un mélange de n-butanol et d'eau dans les conditions décrites ci-dessus et ensuite lavés avec du n-butanol pur, puis avec du cyclohexane.
Rendement : 65% par rapport à la diosmine de départ.
Point de fusion : 184-186°C

Analyse élémentaire :

| | C | H | N |
|---|---|---|---|
| % trouvé | 57,71 | 5,96 | 2,83 |
| % calculé | 57,76 | 5,95 | 3,06 |

Spectre de masse :

Le spectre de masse a été réalisé en désorption ionisation chimique ($NH_3$) ; Température de la source 175°C ; Chauffage du filament 50 à 650 mA à 7 mA/s.
915 m/z [M+H]+ ; 733 m/z ; 607 m/z (100%) ; 326 m/z ; 308 m/z ; 267 m/z ; 181 m/z ; 164 m/z ; 146 m/z ; 129 m/z

L'hémitartrate du rhamnoglucoside-7 de la **[{[(triméthoxy-2,3,4 phényl) méthyl]-4 pipérazinyl-1}-2 oxo-2 éthoxy]**-3' méthoxy-4' hydroxy-5 flavone a été préparé en salifiant la base correspondante en solution dans un mélange de méthanol et d'acétate d'éthyle par une quantité adéquate d'acide 1-tartrique.
Solubilité dans l'eau : 200 g/l.

## EXEMPLE 4

**β glucoside-7 de la [{[(triméthoxy-2,3,4 phényl) méthyl]-4 pipérazinyl-1}-2 oxo-2 éthoxy]-3' méthoxy-4' hydroxy-5 flavone**

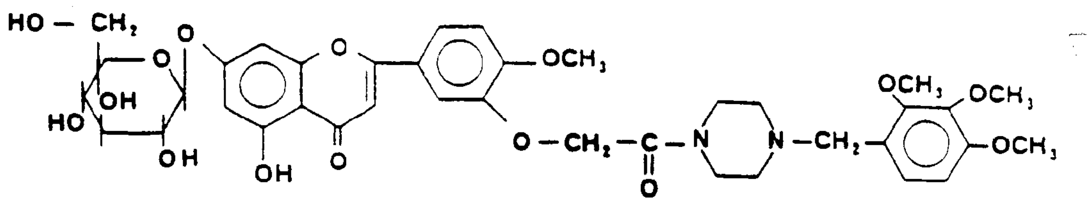

39 mmole de tartrate du rhamnoglucoside-7 de la **[{[(triméthoxy-2,3,4 phényl) méthyl]-4 pipérazinyl-1}-2 oxo-2 éthoxy]**-3' méthoxy-4' hydroxy-5 flavone, composé de l'exemple 3, sont dissous dans un litre d'eau distillée. La solution est ajustée à pH 4 à l'aide d'acide chlorhydrique 0,5 N, portée à 40°C, puis additionnée de 1,2 g de naringinase (Sigma ® N 1385). Après deux heures d'agitation à cette température, la réaction est arrêtée.

Le milieu réactionnel additionné de 200 ml de diméthylformamide est transféré dans une ampoule à décanter et alcalinisé par de l'eau bicarbonatée jusqu'à précipitation nette. L'extraction est réalisée par trois fois 600 ml puis fois 400 ml de n-butanol saturé d'eau. Après décantation, les phases butanoliques sont réunies et évaporées à sec. Le résidu est repris par du méthanol et chauffé à reflux jusqu'à dissolution. La solution ainsi formée est laissée pendant 24 heures à température ambiante. Les cristaux formés sont séparés par filtration et ensuite séchés à 45°C sous vide.
Rendement : 79%.
Point de fusion : 132-134°C

Analyse élémentaire :

| | C | H | N |
|---|---|---|---|
| % trouvé | 56,64 | 5,89 | 3,33 |
| % calculé | 56,54 | 6,03 | 3,47 |

Spectre de masse :

Le spectre a été réalisé avec source FAB mode positif ; Gaz incident Krypton ; Energie 7keV ; Matrice glycérol.
769 m/z [M+H]+ ; 767 m/z 737 m/z ; 607 m/z ; 589 m/z ; 427 m/z; 301 m/z.

Le monophosphate du β glucoside-7 de la [{[(triméthoxy-2,3,4 phényl) méthyl]-4 pipérazinyl-1}-2 oxo-2 éthoxy]-3′ méthoxy-4′ hydroxy-5 flavone a été préparé après addition d'une quantité adéquate d'acide phosphorique dans une solution d'acétone contenant le ß glucoside-7 de la [{[(triméthoxy-2,3,4 phényl) méthyl]-4 pipérazinyl-1}-2 oxo-2 éthoxy]-3′ méthoxy-4′ hydroxy-5 flavone.
Solubilité dans l'eau : 43 g/l.

**EXEMPLE 5**

**[{[(triméthoxy-2,3,4 phényl) méthyl]-4 pipérazinyl-1}-2 oxo-2 éthoxy]-3′ méthoxy-4′ dihydroxy-5,7 flavone**

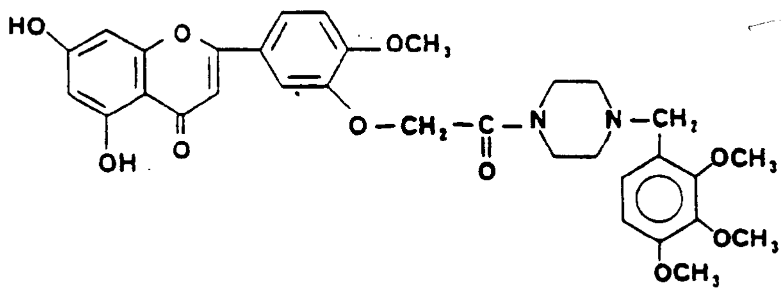

Procédé A

20 mmole du composé de l'exemple 4 sont agités dans 300 ml d'un mélange d'acétone et d'eau (V/V) à température ambiante. On additionne alors 20 ml d'une solution aqueuse normale d'acide phosphorique et 200 ml d'eau. Le mélange est agité au bain-marie jusqu'à dissolution totale puis il est concentré au rotavapor jusqu'à distillation totale de l'acétone. La solution aqueuse résiduelle de pH 3,5 est diluée l'eau pour arriver à un volume final de 1 litre et à pH 3,95, puis portée à 37°C. On ajoute alors 550 mg de β-glucosidase (Sigma ® type II G 8625 à 7,1 μ/mg) et on laisse la réaction à cette température sous agitation pendant 48 heures.

Le milieu réactionnel est porté à pH 7 par addition d'eau bi-carbonatée puis extrait par du n-butanol aqueux (800 ml, 400 ml, 300 ml). Les phases butanoliques réunies sont clarifiées par passage sur célite et après une nouvelle décantation, sont évaporées à sec.

Le résidu est purifié sur colonne de silice (Merck ® 9385) en utilisant comme solvant d'élution un mélange de dichlorométhane et d'acétone (V/V).
Rendement : 77%

Procédé B

STADE A

bis (O-acétyl)-5,7 O-carboxyméthyl-3′ diosmétine

7,5 mmole de l'acide obtenu au Stade B de l'exemple 1 sont solubilisés en chauffant dans 20 ml d'un

mélange de pyridine et d'anhydride acétique (V/V). Le mélange réactionnel est laissé 48 heures à température ambiante, puis on additionne d'eau et on extrait par le dichlorométhane. Après lavage à l'eau, séchage sur sulfate de sodium anhydre et filtration, on récupère après évaporation un résidu, fournissant par cristallisation dans le méthanol le produit attendu pur.
Rendement : 60%

STADE B

[{[(triméthoxy-2,3,4 phényl) méthyl]-4 pipérazinyl-1}-2 oxo-2 éthoxy]-3′ méthoxy-4′ bis acétoxy-5,7 flavone

2 mmole du composé obtenu au stade précédent sont suspendus dans 60 ml de dichlorométhane au bain de glace. On ajoute ensuite 2 mmole de triéthylamine et 2 mmole de chloroformiate d'éthyle. Après 40 minutes à 0°C, on ajoute 2 mmole de [(triméthoxy-2,3,4 phényl) méthyl]-4 pipérazine. Le milieu réactionnel est agité pendant 45 minutes à température ambiante, et ensuite il est lavé à l'eau et évaporé à sec pour obtenir le composé attendu.

STADE C

Le composé obtenu au stade précédent est solubilisé dans 40 ml de diméthylformamide et agité à 100°C sous azote en présence de 6 mmole de carbonate acide de potassium pendant 4 heures. Après refroidissement, le milieu est filtré et évaporé à sec pour donner le composé attendu.
Rendement global : 40%
Point de fusion : 200-202°C

Analyse élémentaire :

| | C | H | N |
|---|---|---|---|
| % trouvé | 63,41 | 5,63 | 4,59 |
| % calculé | 63,36 | 5,65 | 4,62 |

Spectre de masse :

Le spectre de masse a été réalisé en désorption ionisation chimique ($NH_3$) ; Température de la source 160°C ; Intensité du filament 250 μA.
607 m/z [M+H]+ ; 441 m/z ; 427 m/z ; 425 m/z ; 309 m/z ; 307 m/z ; 301 m/z ; 267 m/z ; 181 m/z.

Le méthanesulfonate correspondant a été préparé par salification de la [{[(triméthoxy-2,3,4 phényl) méthyl]-4 pipérazinyl-1}-2 oxo-2 éthoxy]-3′ méthoxy-4′ dihydroxy-5,7 flavone en solution dans un mélange de tétrahydrofuranne et d'eau avec l'acide méthanesulfonique.
Solubilité dans l'eau : 35 g/l.

**EXEMPLE 6**

**[{[(triméthoxy-2,3,4 phényl) méthyl]-4 pipérazinyl-1}-2 oxo-2 éthoxy]-3′ méthoxy-4′ hydroxy-5 (éthoxy-2 oxo-2 éthoxy)-7 flavone**

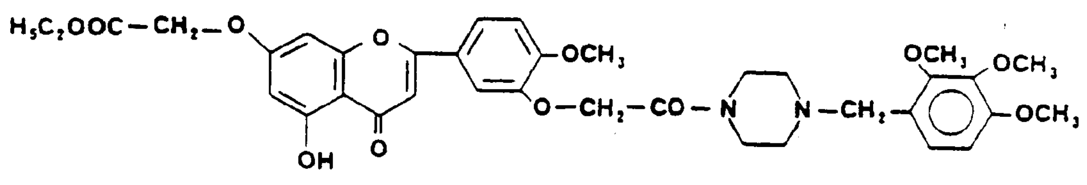

A 5 mmole du composé de l'exemple 5 et 5,25 mmole de carbonate acide de potassium, on ajoute 15 ml de diméthylformamide anhydre sous azote et le mélange est chauffé sous agitation à 110°C. Après dissolution

du composé de l'exemple 5, on ajoute 20 mmole de chloroacétate d'éthyle et la réaction est poursuivie pendant 2 heures. Après refroidissement, le milieu réactionnel est filtré et évaporé à sec. Le résidu est ensuite recristallisé dans l'éthanol pour donner le produit attendu.
Rendement : 60%
Point de fusion : 105-106°C

Analyse élémentaire :

| | C | H | N |
|---|---|---|---|
| % trouvé | 62,03 | 5,90 | 3,96 |
| % calculé | 62,42 | 5,82 | 4,04 |

Spectre de masse :

Le spectre de masse a été réalisé en désorption ionisation chimique ($NH_3$) ; Température de la source 175°C ; Chauffage du filament 50 à 650 mA à 7 mA/s.
693 m/z [M+H]+ ; 511 m/z ; 267 m/z ; 181 m/z

Le phosphate de la [{[(triméthoxy-2,3,4 phényl) méthyl]-4 pipérazinyl-1}-2 oxo-2 éthoxy]-3′ méthoxy-4′ hydroxy-5 (éthoxy-2 oxo-2 éthoxy)-7 flavone a été préparé par addition à une solution hydroacétonique de la base correspondante d'une quantité adéquate de l'acide phosphorique.
Solubilité dans l'eau : 39,5 g/l

## EXEMPLE 7

**Bis [{[(triméthoxy-2,3,4 phényl) méthyl]-4 pipérazinyl-1}-2 oxo-2 éthoxy]-7,3′ hydroxy-5 méthoxy-4′ flavone**

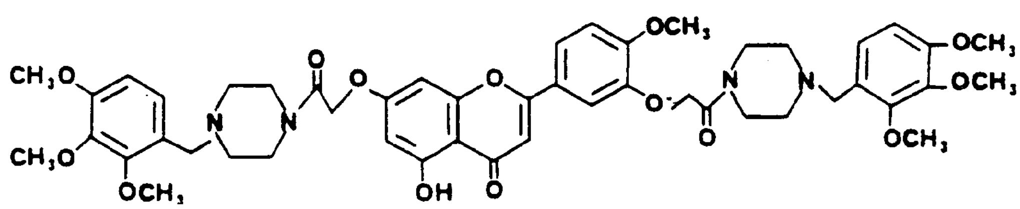

A 5 mmole du composé de l'exemple 5 et 6 mmole de carbonate acide de potassium, on ajoute 15 ml de diméthylformamide anhydre sous azote et agitation à 110°C. Ensuite, on additionne 6 mmole de (chloro-1 acétyl)-1 [(triméthoxy-2,3,4 phényl) méthyl]-4 pipérazine en solution dans le diméthylformamide et on poursuit la réaction pendant 3 heures. Après refroidissement, le milieu réactionnel est filtré, évaporé à sec puis purifié par chromatographie sur colonne de silice (Merck ® 9385) en utilisant comme solvant d'élution un mélange d'acétone et de dichlorométhane (2 : 1 V/V).
Rendement : 60%

Analyse élémentaire :

| | C | H | N |
|---|---|---|---|
| % trouvé | 61,25 | 6,05 | 5,84 |
| % calculé * | 61,30 | 6,33 | 5,96 |

* corrigé 3% $H_2O$

Spectre de masse :

Le spectre de masse a été obtenu avec une source FAB positif ; Gaz incident Krypton ; Energie 7 eV.Matrice Glycérol
913 m/z [M+H]+ ; 733 m/z ; 607 m/z

Le biphosphate de la bis [{[(triméthoxy-2,3,4 phényl) méthyl]-4 pipérazinyl-1}-2 oxo-2 éthoxy]-7,3′ hydroxy-5 méthoxy-4′ flavone a été préparé par salification de la base correspondante en solution dans un mélange d'acétone eau avec l'acide phosphorique.
Solubilité dans l'eau : 221 g/l.

**EXEMPLE 8**

**[{[(triméthoxy-2,3,4 phényl) méthyl]-4 pipérazinyl-1}-2 oxo-2 éthoxy]-7 dihydroxy-5,3′ méthoxy-4′ flavanone**

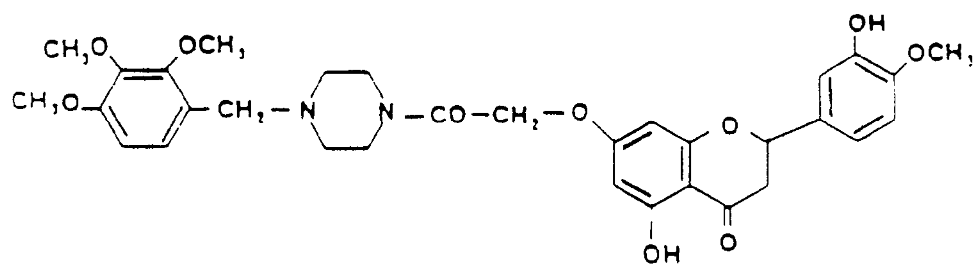

120 mmole d'hespérétine sont agités en présence de 132 mmole de carbonate acide de potassium dans 500 ml de diméthylformamide anhydre à 110°C sous azote. Après dissolution de l'hespérétine, on ajoute 126 mmole de (chloro-1 acétyl)-1 [(triméthoxy-2,3,4 phényl) méthyl]-4 pipérazine en solution dans 100 ml de diméthylformamide et l'on poursuit l'agitation à 110°C pendant 2 heures.

Après refroidissement, le milieu réactionnel est filtré sur verre fritté et évaporé à sec.

Le résidu sec est dissous dans 600 ml de dichlorométhane, et après une heure de repos à température ambiante, est filtré sur célite. Le filtrat est ensuite purifié sur colonne de silice (Amicon ® 84068), en utilisant comme éluant un mélange de dichlorométhane et de méthanol (97,5 : 2,5 V/V).
Rendement : 49%

Analyse élémentaire :

| | C | H | N |
|---|---|---|---|
| % trouvé | 62,78 | 6,07 | 4,54 |
| % calculé | 63,15 | 5,96 | 4,60 |

Spectre de masse :

Le spectre de masse a été obtenu par désorption ionisation chimique ($NH_3$) ; Température de la source 175°C ; Chauffage de filament 50 à 60 mA à 7 mA/s.
609 m/z ; [M+H]+ ; 325 m/z ; 309 m/z ; 303 m/z ; 267 m/z ; 181 m/z

Le méthanesulfonate de la [{[(triméthoxy-2,3,4 phényl) méthyl]-4 pipérazinyl-1}-2 oxo-2 éthoxy]-7 dihydroxy-5,3′ méthoxy-4′ flavanone a été préparé par addition d'une quantité adéquate de l'acide méthanesulfonique à une solution hydroacétonique de la base indiquée ci-dessus.
Solubilité dans l'eau : 1,4 g/l.

## EXEMPLE 9

**Bis [(benzyl-4 piperazinyl-1)-2 oxo-2 éthoxy]-7,3′ hydroxy-5 méthoxy-4′ flavone**

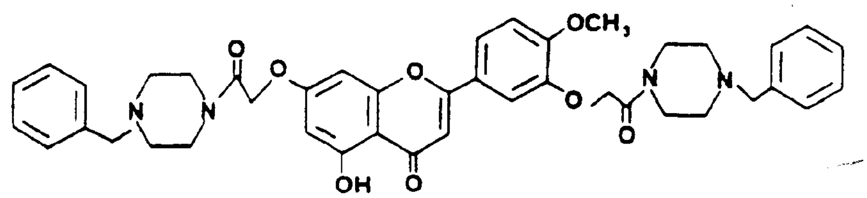

Ce composé a été préparé selon le procédé décrit dans l'exemple 7 mais en utilisant la (chloro-1 acétyl)-1 benzyl-4 pipérazine au lieu de la (chloro-1 acétyl)-1 [(triméthoxy-2,3,4 phényl) méthyl]-4 pipérazine. Rendement : 25% (par rapport à la diosmine)

Analyse élémentaire :

| | C | H | N |
|---|---|---|---|
| % trouvé | 68,65 | 5,95 | 7,20 |
| % calculé | 68,84 | 6,05 | 7,65 |

Spectre de résonance magnétique du proton (400 MHz - solvant $CDCl_3$):
2,5 ppm,m,8H; 3,58 ppm,s,2H; 3,6 ppm,s,2H; 3,7 ppm,m,8H; 4,0 ppm,s,3H; 4,8 ppm,s,2H; 4,87 ppm,s,2H; 6,4 ppm,d,1H; 6,6 ppm,t,2H; 7,0 ppm,d,1H; 7,32 ppm,m,10H; 7,45 ppm,d,1H; 7,6 ppm,d,1H; 12,8 ppm,1H échangeable.

## EXEMPLE 10

**[{[(chloro-2 phényl)méthyl]-4 pipérazinyl-1}-2 oxo-2 éthoxy]-3′ méthoxy-4′ hydroxy-5 (éthoxy-2 oxo-2 éthoxy)-7 flavone**

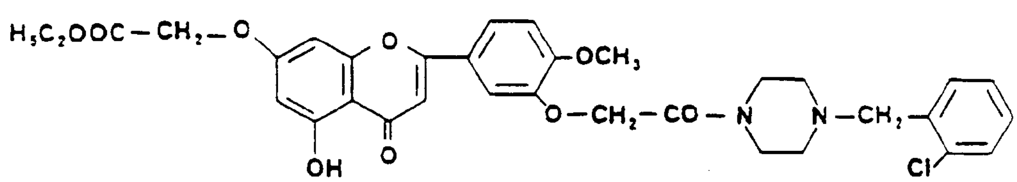

Ce composé a été synthétisé à partir de la [{[(chloro-2 phényl)méthyl]-4 pipérazinyl-1}-2 oxo-2 éthoxy]-3′ méthoxy-4′ dihydroxy-5,7 flavone selon le procédé décrit dans l'exemple 6. Ce dernier composé à été obtenu par alkylation de la diosmétine avec le (chloro-1 acétyl)-1 [(chloro-2 phényl)méthyl]-4 pipérazine. Rendement : 27% (par rapport à la diosmine)

Spectre de résonance magnétique nucléaire du proton (400 MHz - solvant $CDCl_3$):
1,2 ppm,t,3H; 2,5 à 3,58 ppm,m,8H; 3,58 ppm,s,2H; 3,85 ppm,s,3H; 4,2 ppm, q,2H; 4,62 ppm,s,2H; 4,76 ppm,s,2H; 6,28 ppm,d,1H; 6,43 ppm,1H.; 6,45 ppm,s,1H; 6,9 ppm,d,1H; 7,15 à 7,3 ppm,m,4H; 7,38 ppm,d,1H; 7,45 ppm,d,1H; 12,7 ppm,1H échangeable.

**EXEMPLE 11**

**[(benzyl-4 pipérazinyl-1)-2 oxo-2 éthoxy]-5 bis (éthoxy-2 oxo-2 éthoxy)-7,3′ méthoxy-4′ flavone**

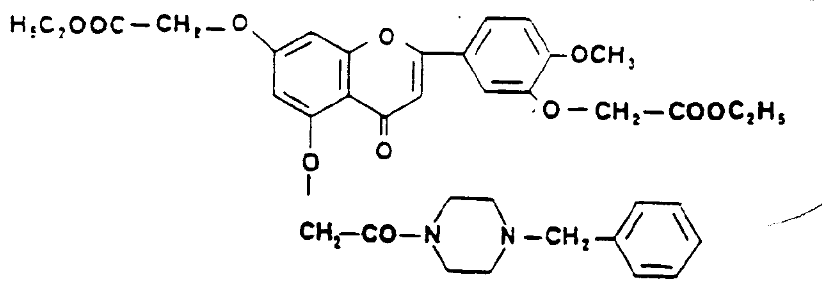

Ce composé a été préparé selon le procédé décrit dans l'exemple 2 par condensation de l'hydroxy-5 bis (éthoxy-2 oxo-2 éthoxy)-7,3′ méthoxy-4′ flavone et de la (chloro-1 acétyl)-1 benzyl-4 pipérazine.
Rendement : 37%

Analyse élémentaire :

| | C | H | N |
|---|---|---|---|
| % trouvé | 64,68 | 5,62 | 3,85 |
| % calculé | 64,53 | 5,85 | 4,07 |

Spectre de résonance magnétique du proton (400 MHz - solvant $CDCl_3$):
1,23 ppm,t,6H; 2,35 ppm,m,4H; 3,55 à 3,70 ppm,m,4H; 3,42 ppm,s,2H; 3,9 ppm,s,3H; 4,22 ppm,q,4H; 4,62 ppm,s,2H; 4,70 ppm,s,2H; 4,80 ppm,s,2H; 6,43 ppm,s,1H; 6,5 ppm,d,2H; 6,92 ppm,d,1H; 7,2 ppm,m,5H; 7,25 ppm,d,1H; 7,48 ppm,d,1H.

**EXEMPLE 12**

**[{[(chloro-4 phényl)méthyl]-4 pipérazinyl-1}-2 oxo-2 éthoxy]-5 bis (éthoxy-2 oxo-2 éthoxy)-7,3′ méthoxy-4′ flavone**

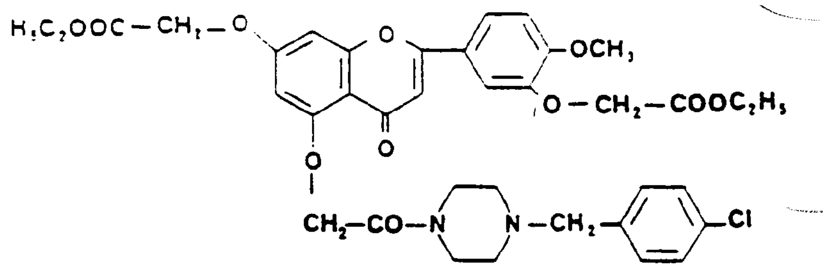

Ce composé a été préparé selon le procédé décrit dans l'exemple 2 par condensation de l'hydroxy-5 bis (éthoxy-2 oxo-2 éthoxy)-7,3′ méthoxy-4′ flavone et de la (chloro-1 acétyl)-1 [(chloro-4 phényl)méthyl]-4 pipérazine.
Rendement : 33%

Analyse élémentaire :

| | C | H | N | Cl |
|---|---|---|---|---|
| % trouvé | 61,19 | 5,20 | 3,65 | 5,23 |
| % calculé | 61,45 | 5,44 | 3,87 | 4,90 |

Spectre de masse :

Le spectre de masse a été obtenu par désorption ionisation chimique ($NH_3$) ; Température de la source 150°C ; Energie 70eV.

723 m/z [M+H]+ ; 637 m/z; 597 m/z; 513 m/z; 485 m/z; 473 m/z; 269 m/z; 253 m/z; 239 m/z; 211 m/z; 142 m/z; 125 m/z

Spectre de résonance magnétique nucléaire du proton (400 MHz - solvant $CDCl_3$):

1,25 ppm,t,6H; 2,35 à 3,55 ppm,m,4H; 2,30 à 3,70 ppm,m,4H; 3,45 ppm,s,2H; 3,9 ppm,s,3H; 4,22 ppm,q,4H; 4,60 ppm,s,2H; 4,70 ppm,s,2H; 4,80 ppm,s,2H; 6,45 ppm,s,1H; 6,5 ppm,d,2H; 6,92 ppm,d,1H; 7,3 ppm,d,1H; 7,3 ppm,m,4H; 7,48 ppm,d,1H.

## EXEMPLE 13

**[{[(méthyl-4 phényl)méthyl]-4 pipérazinyl-1}-2 oxo-2 éthoxy]-5 bis (éthoxy-2 oxo-2 éthoxy)-7,3′ méthoxy-4 flavone.**

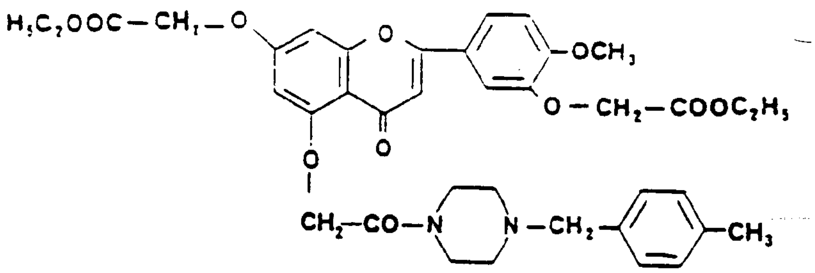

Ce composé a été préparé selon le procédé décrit dans l'exemple 2 par condensation de l'hydroxy-5 bis (éthoxy-2 oxo-2 éthoxy)-7,3′ méthoxy-4′ flavone et de la (chloro-1 acétyl)-1 [(méthyl-4 phényl) méthyl]-4 pipérazine.

Rendement : 25%

Analyse élémentaire :

| | C | H | N |
|---|---|---|---|
| % trouvé | 64,18 | 5,67 | 3,66 |
| % calculé | 64,95 | 6,02 | 3,99 |

Spectre de résonance magnétique du proton (400 MHz - solvant $CDCl_3$):

1,30 ppm,t,6H; 2,30 ppm,s,3H; 2,40 à 3,60 ppm,m,4H; 2,40 à 3,75 ppm,m,4H; 3,45 ppm,s,2H; 3,95 ppm,s,3H; 4,30 ppm,q,4H; 4,70 ppm,s,2H; 4,75 ppm,s,2H; 4,85 ppm,s,2H; 6,50 ppm,s,1H; 6,60 ppm,d,2H; 7,00 ppm,d,1H; 7,1 ppm,m,4H; 7,30 ppm,d,1H; 7,55 ppm,d,1H.

**EXEMPLE 14**

**{(phényl-4 pipérazinyl-1)-2 oxo-2 éthoxy}-5 bis (éthoxy-2 oxo-2 éthoxy)-7,3′ méthoxy-4′flavone**

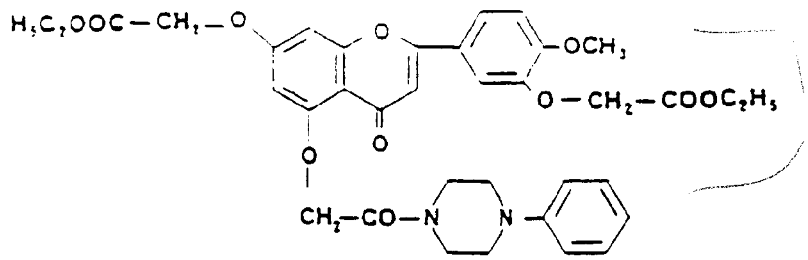

Ce composé a été préparé selon le procédé décrit dans l'exemple 2 par condensation de l'hydroxy-5 bis (éthoxy-2 oxo-2 éthoxy)-7,3′ méthoxy-4′ flavone et de la (chloro-1 acétyl)-1 phényl-4 pipérazine.
Rendement : 30%

Analyse élémentaire :

| | C | H | N |
|---|---|---|---|
| % trouvé | 63,79 | 5,37 | 4,09 |
| % calculé | 64,09 | 5,68 | 4,15 |

Spectre de résonance magnétique nucléaire du proton (400 MHz - solvant $CDCl_3$):
1,3 ppm,t,6H; 3,15 à 3,75 ppm,m,4H; 3,15 à 3,95 ppm,m,4H; 3,95 ppm,s,3H; 4,3 ppm,q,4H; 4,6 ppm,s,2H; 4,65 ppm,s,2H; 4,90 ppm,s,2H; 6,50 ppm,d,1H; 6,55 ppm,d,1H; 6,65 ppm,d,1H; 6,85 à 7,20 ppm,m,5H; 7,0 ppm,d,1H; 7,3 ppm,d,1H; 7,55 ppm,d,1H.

**EXEMPLE 15**

**[{[(chloro-4 phényl) méthyl]-4 pipérazinyl-1}-2 oxo-2 éthoxy]-7 hydroxy-5 méthoxy-4′ (éthoxy-2 oxo-2 éthoxy)-3′ flavone**

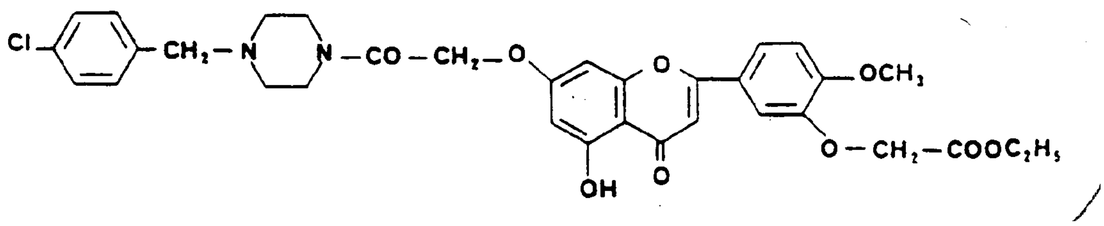

Ce composé a été préparé par condensation de la O-éthoxycarbonyl méthyl-3′ diosmétine et de (chloro-1 acétyl)-1 [(chloro-4 phényl)méthyl]-4 pipérazine selon le procédé décrit dans l'exemple 1.
Rendement : 84%

Analyse élémentaire :

| | C | H | N | Cl |
|---|---|---|---|---|
| % trouvé | 62,02 | 5,16 | 4,38 | 5,62 |
| % calculé | 62,22 | 5,22 | 4,40 | 5,56 |

Spectre de résonance magnétique du proton (400 MHz - solvant $CDCl_3$):
1,23 ppm,t,3H; 2,45 à 3,60 ppm,m,4H; 2,45 à 3,65 ppm,m,4H; 3,50 ppm,s,2H; 3,85 ppm,s,3H; 4,22 ppm,q,2H; 4,8 ppm,s,4H; 6,35 ppm,d,1H; 6,55 ppm,s,1H; 6,55 ppm,d,1H; 7,30 ppm,m,4H; 7,35 ppm,d,1H; 7,60 ppm,d,1H; 12,8 ppm,1H échangeable.

## EXEMPLE 16

**[(benzyl-4 pipérazinyl-)-2 oxo-2 éthoxy]-7 hydroxy-5 méthoxy-4′ (éthoxy-2 oxo-2 éthoxy)-3′ flavone**

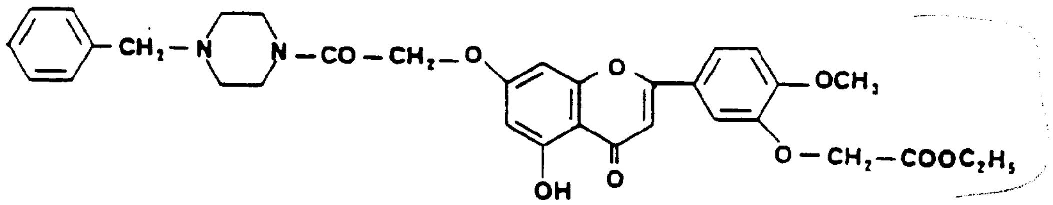

Ce composé a été préparé par condensation de la O-éthoxycarbonyl méthyl-3′ diosmétine et de (chloro-1 acétyl)-1 benzyl-4 pipérazine selon le procédé décrit dans l'exemple 1.
Rendement : 86%

Analyse élémentaire :

| | C | H | N |
|---|---|---|---|
| % trouvé | 65,44 | 5,52 | 4,91 |
| % calculé | 65,77 | 5,69 | 4,65 |

Spectre de résonance magnétique du proton (400 MHz - solvant $CDCl_3$):
1,30 ppm,t,3H; 2,5 à 3,55 ppm,m,4H; 2,5 à 3,65 ppm,m,4H; 3,55 ppm,s,2H; 4,0 ppm,s,3H; 4,3 ppm,q,2H; 4,8 ppm,s,4H; 6,35 ppm,d,1H; 6,55 ppm,d,1H; 6,55 ppm,s,1H; 7,0 ppm,d,1H; 7,3 ppm,m,5H; 7,35 ppm,d,1H; 7,6 ppm,d1H; 12,8 ppm,1H échangeable.

## EXEMPLE 17

**Rhamnoglucoside-7 de la [(benzyl-4 pipérazinyl-1)-2 oxo-2 éthoxy]-3′ méthoxy-4′ hydroxy-5 flavone**

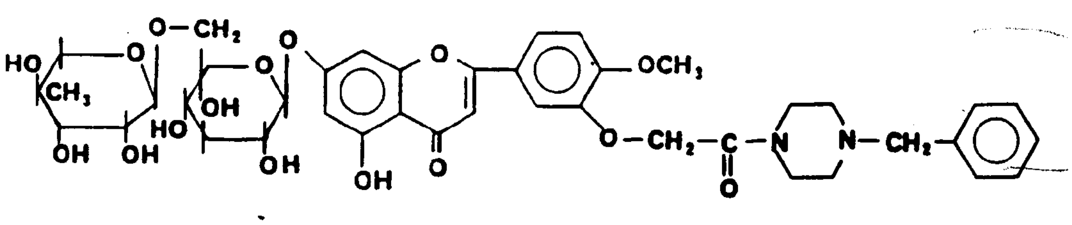

Ce composé a été préparé à partir de diosmine et de (chloro-1 acétyl)-1 benzyl-4 pipérazine selon le procédé décrit dans l'exemple 3.
Rendement : 70%
Point de fusion : 214-217°C.

Analyse élémentaire :

| | C | H | N |
|---|---|---|---|
| % trouvé | 59,63 | 5,98 | 3,38 |
| % calculé | 59,70 | 5,87 | 3,40 |

Spectre de masse :

Les spectres ont été réalisés avec source FAB mode positif et négatif; Gaz incident Krypton; Energie 8 KeV; Matrice Glycerol - Thioglycerol (1/1)
FAB+ : 825 m/z [M+H]+; 823 m/z; 733 m/z; 679 m/z; 661 m/z; 517 m/z; 91 m/z.
FAB- : 824 m/z [M].-; 823 m/z; 809 m/z; 663 m/z; 621 m/z ; 607 m/z; 515 m/z; 299 m/z.

**EXEMPLE 18**

**Rhamnoglucloside-7 de la [{[(chloro-2 phényl) méthyl]-4 pipérazinyl-1}-2 oxo-2 éthoxy]-3′ méthoxy-4′ hydroxy-5 flavone**

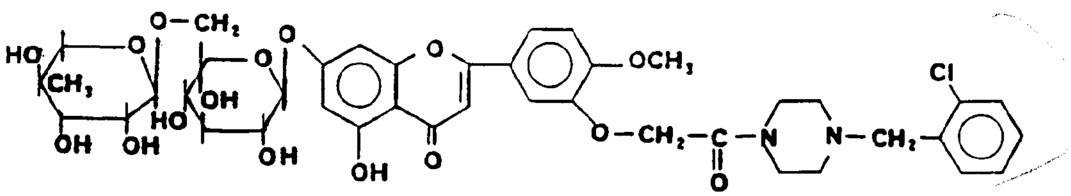

Ce composé a été préparé à partir de la diosmine et de (chloro-1 acétyl)-1 [(chloro-2 phényl)méthyl]-4 pipérazine selon le procédé décrit dans l'exemple 3.
Rendement : 64%
Point de fusion : 200-222°C

Analyse élémentaire :

| | C | H | N | Cl |
|---|---|---|---|---|
| % trouvé | 57,42 | 5,42 | 3,14 | 4,29 |
| % calculé | 57,31 | 5,51 | 3,26 | 4,13 |

Spectre de masse :
Les spectres ont été réalisés avec source FAB mode positif et négatif; Gaz Incident Krypton; energie 8 KeV; Matrice Glycerol - Thioglycerol (1/1)
FAB+ : 859 m/z [M+H]+; 713 m/z; 551 m/z; 147 m/z; 125 m/z;
FAB- : 858 m/z [M].-; 857 m/z; 607 m/z; 549 m/z; 299 m/z ; 163 m/z;

**EXEMPLE 19**

**Rhamnoglucloside-7 de la [{[(chloro-4 phényl) méthyl]-4 pipérazinyl-1}-2 oxo-2 éthoxy]-3′ méthoxy-4′ hydroxy-5 flavone**

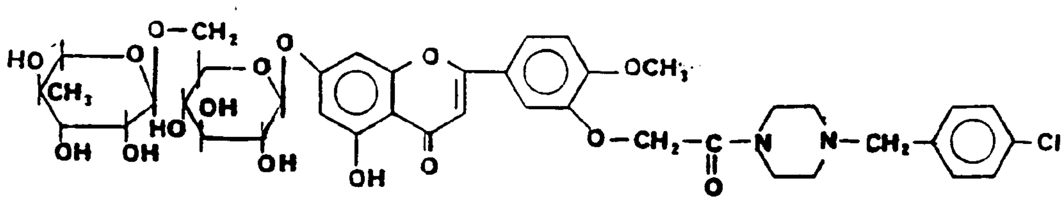

Ce composé a été préparé selon le procédé décrit dans l'exemple 3 à partir de la diosmine et de (chloro-1 acétyl)-1 [(chloro-4 phényl)méthyl]-4 pipérazine.
Rendement : 30%

Spectre de masse :
Les spectres ont été réalisés avec source FAB mode positif et négatif; Gaz Incident Krypton; Energie 8 KeV; Matrice Glycerol - Thioglycerol (1/1).
FAB+ : 859 m/z [M+H]+; 857 m/z; 551 m/z;
FAB- : 858 m/z [M].-; 549 m/z.

**EXEMPLE 20**

**Rhamnoglucloside-7 de la [{[(méthyl-4 phényl) méthyl]-4 pipérazinyl-1}-2 oxo-2 éthoxy]-3′ méthoxy-4′ hydroxy-5 flavone**

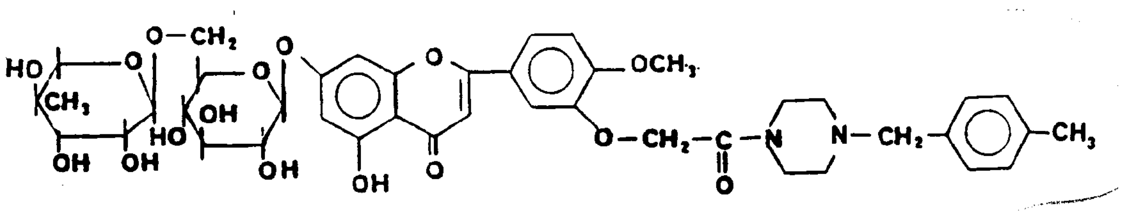

Ce composé a été préparé selon le procédé décrit dans l'exemple 3 à partir de la diosmine et de (chloro-1 acétyl)-1 [(méthyl-4 phényl) méthyl]-4 pipérazine.
Rendement : 56%

Spectre de masse :

Les spectres ont été réalisés avec source FAB mode positif et négatif; Gaz Incident Krypton; Energie 8 KeV; Matrice diéthanolamine.
FAB+ : 838 m/z [M+H]+; 821 m/z; 421 m/z; 316 m/z; 211 m/z; 105 m/z;
FAB- : 838 m/z [M].-; 837 m/z; 529 m/z; 209 m/z.

## EXEMPLE 21

**Rhamnoglucoside-7 de la [(phényl-4 pipérazinyl-1)-2 oxo-2 éthoxy]-3′ méthoxy-4′ hydroxy-5 flavone**

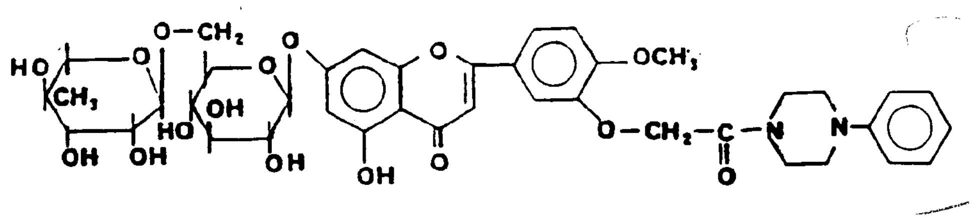

Ce composé a été préparé par condensation de la diosmine avec la (chloro-1 acétyl)-1 phényl-4 pipérazine.
Rendement : 59%
Point de fusion : 218-221°C

Analyse élémentaire :

| | C | H | N |
|---|---|---|---|
| % trouvé | 58,69 | 5,33 | 3,46 |
| % calculé | 59,25 | 5,75 | 3,45 |

Spectrométrie de masse:

Les spectres ont été réalisés avec source FAB mode positif et négatif; Gaz Incident Krypton; Energie 8 KeV; Matrice Glycérol -Thioglycérol (1/1)
$FAB^+$ : 811 m/z $[M+H]^+$;
$FAB^-$ : 809 m/z; 795m/z;

## ETUDE PHARMACOLOGIQUE

## EXEMPLE 22

**Oedème du croton de l'oreille de souris**

Cet essai consiste à inflammer les oreilles de souris avec une solution irritante à base d'huile de croton et à tester l'effet préventif des composé à examiner administrés per os.

Les essais ont été réalisés sur des lots d'au moins 8 souris mâles $CD_1$ (Charles River) de poids moyen 18-20 g. Les composés de l'invention ont été administrés par gavage avec une sonde, en prise unique, à raison de 0,1 ml de liquide par 10 g de souris. La souris a été privée de nourriture mais pas de boisson, 2 heures avant le gavage et jusqu'après l'irritation.

Trente minutes après le gavage, chaque souris a été légèrement anesthésiée à l'éther. 10 µl de la solution irritante ont été ensuite déposés à l'aide d'une pipette Eppendorf sur la surface cutanée du pavillon de l'oreille droite ; l'oreille gauche n'ayant subi aucun traitement, a servi comme témoin. Ensuite, l'animal a été replacé dans la cage et sacrifié six heures plus tard. Les oreilles ont été sectionnées en suivant l'arête cartilagineuse et immédiatement pesées.

Pour apprécier l'activité des produits, l'augmentation moyenne de poids des oreilles des lots traités (m) est comparée par le test t de Student à celle du lot témoin (mt) non traité. L'indice d'activité curative est évalué par calcul du pourcentage d'inhibition (I) selon la formule :

$$I = \frac{mt - m}{mt} \times 100$$

Les composés de l'invention ont montré un effet inhibiteur significatif vis-à-vis de la constitution de l'oedème. Les résultats de l'essai sont indiqués dans le Tableau I.

TABLEAU I

| COMPOSE | DOSE mg.kg-1 | I |
|---|---|---|
| Exemple 2 (chlorhydrate) | 166 | - 34% ($p < 0.05$) |
| Exemple 3 (hemitartrate) | 200 | - 26% ($p < 0.01$) |
| Exemple 4 (monophosphate) | 173 | - 28% ($p < 0.05$) |
| Exemple 6 (monophosphate) | 160 | - 35% ($p < 0.01$) |

## EXEMPLE 23

### Inhibition de la réaction de dégradation de l'AMP cyclique par la phosphodiestérase

L'extrait enzymatique nécessaire pour cette étude est préparé à partir du cerveau de rat.

Après décapitation, le cerveau est prélevé, pesé et congelé une demi-heure à -70°C. Ensuite la préparation, maintenue dans la glace, est diluée et homogénéisée dans du glycérol 50% contenant 50 mmole de tris-(hydroxyméthyl)-aminométhane et 50 mmole de sulfate de magnésium, à raison de 5 ml par gramme de cerveau. La préparation est ensuite centrifugée. Le surnageant est reparti en aliquotes et conservé à 4°C.

Lors de l'étude, l'extrait enzymatique est incubé en présence d'AMP cyclone (Boehringer ® Ref. 102300), de phosphatase alcaline (Boehringer ® Ref. 108138), d'adénosine déaminase (Boehringer ® Ref. 102091) et des quantités croissantes des composés de l'invention. La cinétique de la dégradation de l'AMP cyclique est suivie par spectrophotométrie à 25°C. Comme le blanc est utilisé un mélange dépourvu de phosphatase alcaline et d'adénosine déaminase. La mesure de la densité optique se fait à 265 nm. La concentration des composés de l'invention entraînant une inhibition de 50% de la réaction enzymatique ($IC_{50}$) est calculée. Deux substances de référence, la diosmine et la théophylline, sont étudiées selon la même méthodologie.

Les résultats de cette étude indiqués dans le Tableau II ont montré que les produits de l'invention sont des inhibiteurs très puissants de la réaction de dégradation de l'AMP cyclique par la phosphodiestérase et confirment l'intérêt de leurs emplois en thérapeutique. En effet, il est connu que la libération des médiateurs biochimiques de l'inflammation tels que histamine, leucotriènes, enzymes lysosomiales et radicaux libres est inhibée par l'augmentation de la concentration intracellulaire d'AMP cyclique. (Kaliner M, Austen K.F. "Bioch.Pharmacol",(1974)23,p. 763-771 et Lehmeyer J.E.Johnston R.B.Jr. "Clin.Immunol.Immunopathol",(1978),9, p. 482-490). Les produits de l'invention en inhibant la réaction de dégradation de l'AMP cyclique augmentent sa concentration intracellulaire et contribuent à la diminution de la libération des médiateurs biochimiques de l'inflammation. A noter que les produits de l'invention possèdent une activité inhibitrice beaucoup plus importante par rapport aux produits utilisés habituellement comme référence : théophylline et diosmine.

TABLEAU II

| COMPOSE | $IC_{50}$ (M) |
|---|---|
| Exemple 2 | 2,0 x $10^{-5}$ |
| Exemple 3 | 2,5 x $10^{-5}$ |
| Exemple 7 | 1,0 x $10^{-5}$ |
| Exemple 8 | 5,7 x $10^{-5}$ |
| DIOSMINE | 10,0 x $10^{-5}$ |
| THEOPHYLLINE | 100,0 x $10^{-5}$ |

## EXEMPLE 24

### Evaluation de l'activité pharmocodynamique vis-à-vis des radicaux libres oxygénés produits par les polymorphonucléaires

L'éventuelle activité pharmacodynamique des composés de l'invention vis-à-vis de la production de radicaux libres par les polymorphonucléaires a été recherchée in vitro. Le modèle cellulaire utilisé était des leucocytes polymorphonucléaires de rat. Ce modèle est capable de produire des radicaux libres d'une façon contrôlable. La production de radicaux libres a été quantifiée par mesure de la chemiluminescence qui est proportionnelle à leur quantité.

Des quantités de 2 x $10^6$ cellules de leucocytes polymorphonucléaires isolés par le sang de rat hépariné sont mélangées avec des solutions de concentrations croissantes des composés de l'invention ($2.10^{-4}$ M à $2.10^{-8}$ M). Ensuite, sont ajoutées successivement des quantités adéquates d'une solution de Hanks, de zymosan ® (Sigma 24250) et de luminol ® (Merck 82007). Les solutions sont gardées à l'abri de la lumière et transférées rapidement dans un compteur à scintillation liquide. Dans l'essai "blanc" les polymorphonucléaires sont remplacés par un volume équivalent de solution de Hanks et dans l'essai "témoin", la solution des "composés à tester" est remplacée par une solution de Hanks. La même étude a été réalisée avec un produit de référence : la diosmine.

Le pourcentage d'inhibition de la production de radicaux libres a été calculée par la formule suivante :

$$\%\,\text{d'inhibition} = 100\% - \left| \frac{\text{Cl témoin} - \text{Cl composés}}{\text{Cl témoin} - \text{Cl blanc}} \right|$$

Cl témoin : Chemiluminescence maximum d'essai "témoin"
Cl composés : Chemiluminescence maximum d'essai "composés à tester"
Cl blanc : Chemiluminescence maximum d'essai "blanc"

Les concentrations des composés de l'invention et de la diosmine, déterminant une inhibition de 50% ($IC_{50}$) de la réaction de chemiluminescence sont données dans le tableau III.

Il est connu que l'accumulation de radicaux libres oxygénés dans le milieu extracellulaire constitue une menace à l'intégrité cellulaire et tissulaire (Bruce A et col. Lab.Invest.,(1982),47,(5),p.412-426). En effet, les métabolites de l'oxygène sont impliqués dans les réactions d'inflammation tissulaire tant au niveau de la réaction vasculaire qu'à celui des atteintes tissulaires et cellulaires.

Les produits de l'invention en inhibant la formation des radicaux libres oxygénés préviennent la constitution et le maintien de l'oedème qui est une manifestation de l'insuffisance veineuse. (Phlebology 85,Eds D.Negus et G.Jantet,1986,John Libley Lon.Par).

TABLEAU III

| COMPOSE | $IC_{50}$ |
|---|---|
| DIOSMINE | $40.10^{-6}$ M |
| EXEMPLE 2 | $4.10^{-6}$ M |
| EXEMPLE 6 | $10.10^{-6}$ M |
| EXEMPLE 8 | $15.10^{-6}$ M |

**EXEMPLE 25**

**Comprimés enrobés dosés à 400 mg de Rhamnoglucoside-7 de la [{(triméthoxy-2,3,4 phényl) méthyl]-4 pipérazinyl-1}-2 oxo-2 éthoxy]-3′ méthoxy-4′ hydroxy-5 flavone (R.T.M.P.M.P.F.).**

| | |
|---|---|
| R.T.M.P.M.P.F. | 400,000 mg |
| Carboxyméthylamidon sodique | 27,000 mg |
| Cellulose microcristalline | 62,000 mg |
| Cire d'abeille blanche | q.s.p. cirer |
| Gélatine | 31,000 mg |
| Glycérol | 0,426 mg |
| Hydroxypropylméthylcellulose | 7,078 mg |
| Laurylsulfate de sodium | 0,034 mg |
| Oxyde de fer jaune | 0,166 mg |
| Oxyde de fer rouge | 0,055 mg |
| Oxyde de titane | 1,362 mg |
| Polyoxyéthylène-glycol 6000 | 0,453 mg |
| Stéarate de magnésium | 4,426 mg |
| Talc | 6,000 mg |

pour un comprimé enrobé.

## Revendications

**Revendications pour les Etats contractants suivants: AT, BE, CH, DE, FR, GB, IT, LI, LU, NL, SE**

1. Composés de formule I :

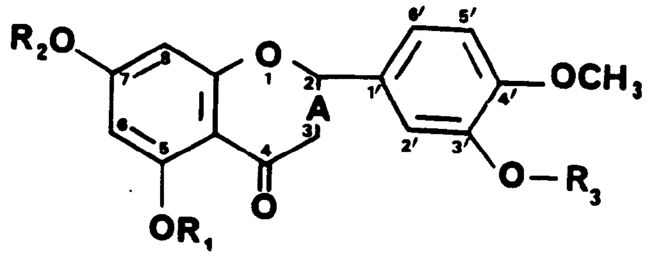

(I)

dans laquelle :

– A représente une simple ou une double liaison,

– $R_1$, $R_3$ identiques ou différents représentant chacun un atome d'hydrogène, un radical alcoxy carbonyl méthylène de formule $-CH_2CO_2R_4$ (dans laquelle $R_4$ représente un radical alkyle de 1 à 5 atomes de carbone) ou un radical de formule W :

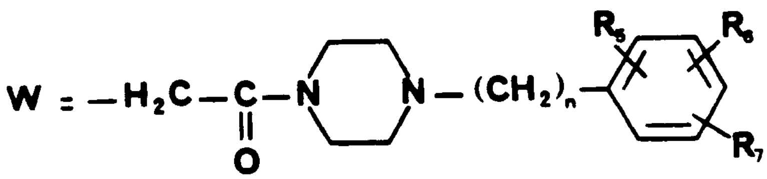

(dans laquelle n est égal à 0 ou 1 et $R_5$, $R_6$, $R_7$ identiques ou différents représentent chacun un atome d'hydrogène ou d'halogène, un radical hydroxyle, un radical trifluorométhyle, un radical alkyle inférieur renfermant de 1 à 5 atomes de carbone ou un radical alcoxy renfermant de 1 à 5 atomes de carbone),

– $R_2$ représente un atome d'hydrogène, un radical alcoxy carbonyl méthylène de formule $-CH_2CO_2R_4$, un radical de formule W, ou une molécule de β-glucose ou rutinose liée à l'oxygène auquel elle est attachée avec une liaison osidique,

à condition toutefois qu'au moins ou $R_1$, ou $R_2$, ou $R_3$ représente toujours un radical de formule W,

et leurs sels d'addition à un acide minéral ou organique pharmaceutiquement acceptable.

2. Le rhamnoglucoside-7 de la [{[(triméthoxy-2,3,4 phényl) méthyl]-4 pipérazinyl-1}-2 oxo-2 éthoxy]-3′ méthoxy-4′ hydroxy-5 flavone, composé répondant à la formule I selon la revendication 1, et ses sels d'addition avec un acide minéral ou organique, pharmaceutiquement acceptable.

3. La [{[(triméthoxy-2,3,4 phényl) méthyl]-4 pipérazinyl-1}-2 oxo-2 éthoxy]-3′ méthoxy-4′ dihydroxy-5,7 flavone, composé répondant à la formule I selon la revendication 1, et ses sels d'addition avec un acide minéral ou organique pharmaceutiquement acceptable.

4. Procédé de préparation des composés de formule I, selon la revendication 1, caractérisé en ce que :

soit :

l'on fait réagir la diosmine, composé de formule II :

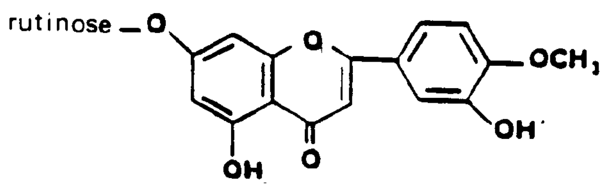

(II)

avec un chlorure d'alkyle de formule III renfermant les composés de formules générales IIIa et IIIb :

$ClCH_2CO_2R_4$ IIIa

$Cl\ W$ IIIb

(III)

dans lesquels $R_4$ et W ont les mêmes significations que pour la formule I, selon la revendication 1, dans un solvant organique azoté polaire, en présence d'un sel minéral acide de métal alcalin à une température comrpise entre 80°-120°,

pour obtenir

- <u>ou</u>

avec les composés de formule IIIa, les composés de formule générale IV :

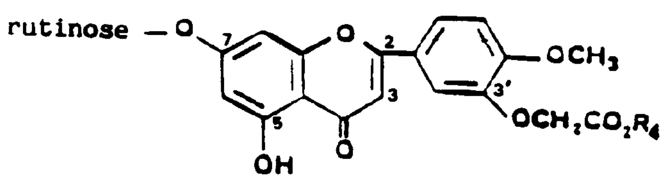

(IV)

dans laquelle $R_4$ a la signification indiquée pour la formule I, selon la revendication 1,

que l'on soumet à une hydrolyse acide en présence d'un acide minéral concentré et à une température comprise entre 35°-55°C pour former le composé de formule V :

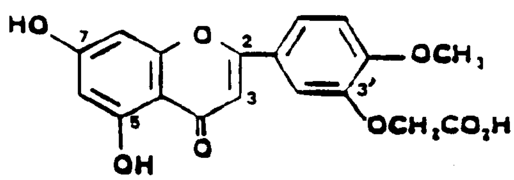

(V)

lequel ensuite :

- soit :

que l'on soumet à une acétylation à l'aide de l'anhydride acétique dans un solvant organique basique et à une température ambiante pour former le composé de formule VI :

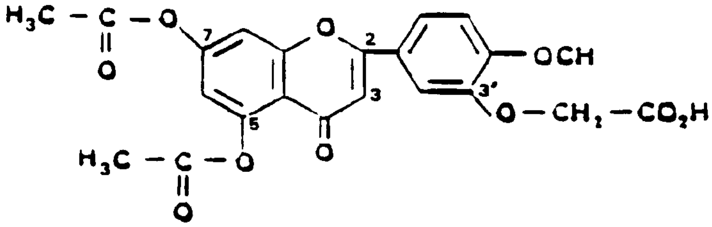

(VI)

que l'on fait réagir avec un dérivé de la pipérazine de formule générale VII :

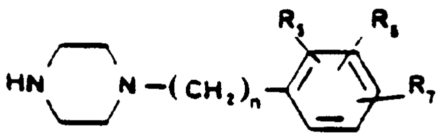

(VII)

dans laquelle n, $R_5$, $R_6$, $R_7$ ont la même signification que pour la formule I, selon la revendication 1, en présence d'une amine tertiaire de petit poids moléculaire et de chloroformiate d'éthyle, à une température comprise entre 0°-20°C pour former les composés de formule générale VIII :

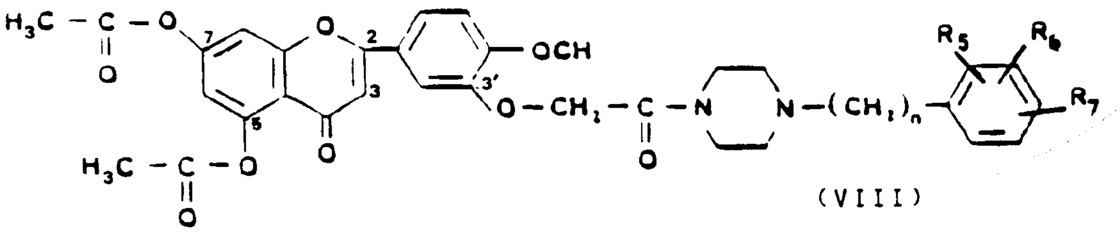

(VIII)

dans laquelle n, $R_5$, $R_6$, $R_7$ ont la signification indiquée, pour la formule I, selon la revendication 1,

que l'on soumet ensuite à une désacétylation en solution dans le diméthylformamide en présence d'un sel minéral acide d'un métal alcalin à une température comprise entre 80°-120°C, pour former les composés de formule générale $I_A$ :

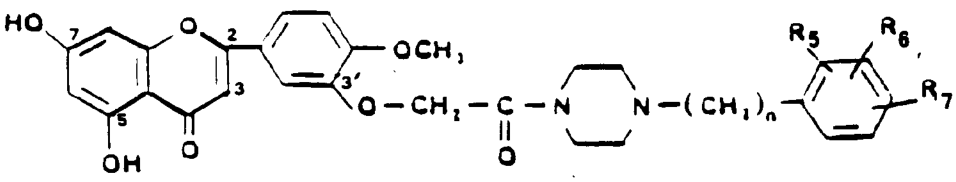

($I_A$)

dans laquelle la signification de n, $R_5$, $R_6$ et $R_7$ reste identique à celle donnée pour la formule I, selon la revendication 1,

que l'on condense avec un composé de formule générale III pour former des composés de formule générale $I_B$ :

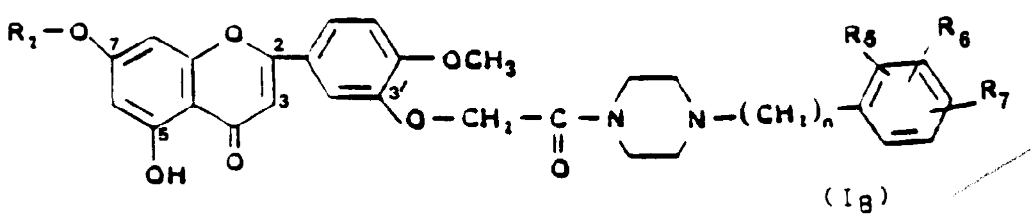

($I_B$)

dans laquelle n, $R_5$, $R_6$ et $R_7$ ont la signification indiquée ci-dessus et $R_2$ représente un radical alcoxy carbonyl méthylène de formule $-CH_2CO_2R_4$ ou un radical de formule W, selon la revendication 1,

- soit :

que l'on estérifie avec un alcool primare ou secondaire contenant de 1 à 5 atomes de carbone, en milieu anhydre à une température comprise entre 80°-110°C et un présence de l'acide tosylique pour former les composés de formule générale IX :

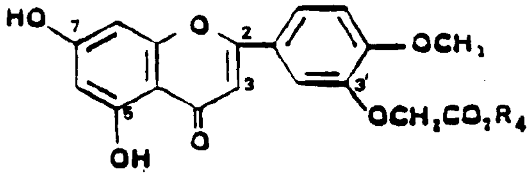

(IX)

dans laquelle $R_4$ a la même signification que pour la formule IV, que l'on fait réagir

■ ou avec un chlorure d'alkyle de formule générale $III_B$ pour former les composés de fomule générale $I_C$ :

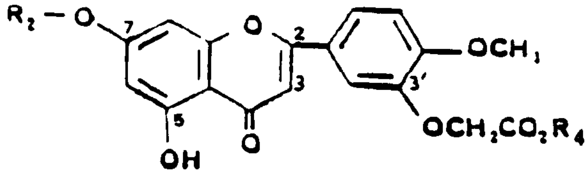

($I_C$)

dans laquelle $R_2$ représente un radical de formule W, selon la revendication 1,

■ ou avec un chlorure d'alkyle de formule $III_A$ pour former les composés de formule générale X :

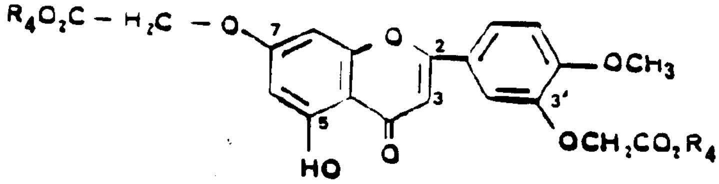

(X)

dans laquelle $R_4$ a la signification indiquée ci-dessus pour la formule I, selon la revendication 1,

que l'on condense avec un composé de formule générale $III_B$ pour obtenir les composés de formule générale $I_D$ :

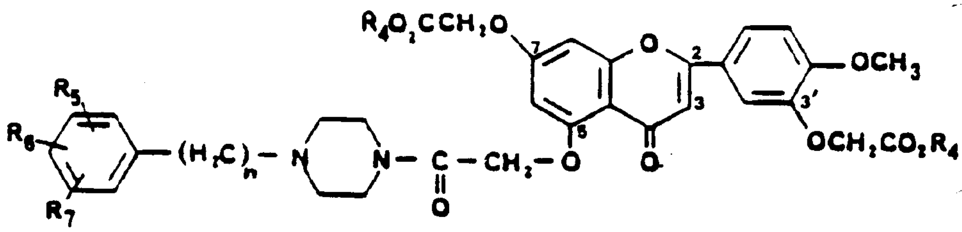

($I_D$)

dans laquelle la signification de n, $R_4$, $R_5$, $R_6$ et $R_7$ reste identique à celle donnée, pour la formule I, selon la revendication 1,

- ou

avec les composés de formule $III_B$, les composés de fomule générale $I_E$,

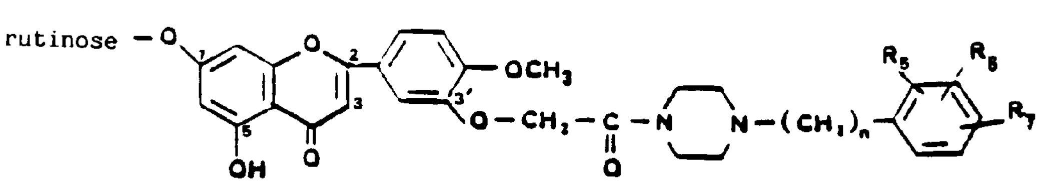

($I_E$)

dans laquelle n, $R_5$, $R_6$, $R_7$ ont la signification indiquée pour la formule I, selon la revendication 1,

que l'on soumet ensuite à une hydrolyse enzymatique à l'aide de naringinase pour former les composés de formule générale $I_F$ :

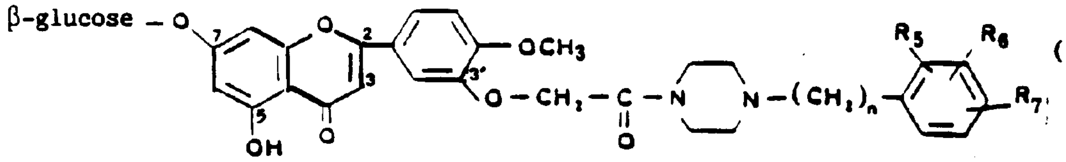

($I_F$)

dans laquelle n, $R_5$, $R_6$ et $R_7$ ont la signification indiquée ci-dessus,

lesquels ensuite,

que l'on soumet à une hydrolyse enzymatique à l'aide de la β-glucosidase pour former les composés de formule générale $I_A$,

que l'on condense ensuite avec un composé de formule générale III pour former les composés de formule générale $I_B$,

soit :

l'on fait réagir un composé de formule générale XI :

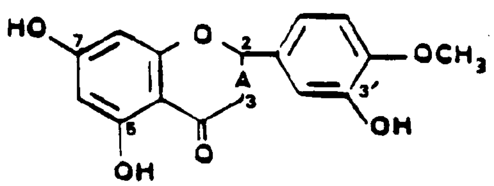

(XI)

dans laquelle A a la même signification que pour la formule I, selon la revendication 1, avec une quantité adéquate des composés de formule générale $III_B$,

pour former les composés de formule générale $I_G$ :

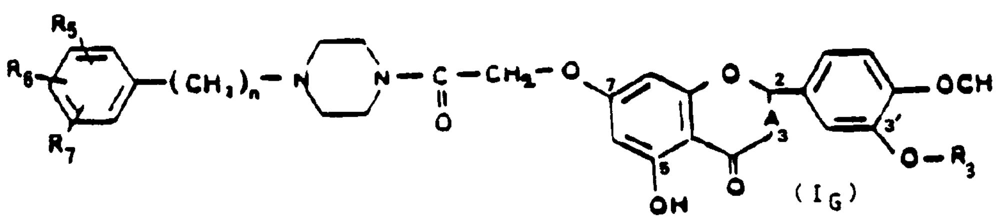

($I_G$)

dans laquelle n, A, $R_5$, $R_6$ et $R_7$ ont la signification indiquée dans la formule I, selon la revendication 1, et $R_3$ représente un atome d'hydrogène quand la quantité des composés de formule générale $III_B$ utilisée est environ équimolaire, ou un radical de formule W selon la revendication 1, quand l'alkylation est effectuée avec une quantité de chlorure d'alkyle de formule générale $III_B$ au moins double,

et que l'on salifie ensuite, si l'on désire, les composés des formules $I_A$ à $I_G$, qui forment l'ensemble des composés de formule I, selon la revendication 1, avec un acide organique ou minéral pharmaceutiquement acceptable.

5. Composition pharmaceutique contenant comme principe actif un composé selon l'une quelconque des revendications 1 à 3, en association ou en mélange avec un excipient ou un véhicule inerte, non toxique, pharmaceutiquement acceptable.

6. Composition pharmaceutique selon la revendication 5 renfermant le principe actif à la dose de 50 à 500 mg.

7. Composition pharmaceutique selon les revendications 5 et 6 renfermant comme principe actif au moins un composé selon les revendications 1 à 3 utilisable pour le traitement des affections vasculaires telles que l'insuffisance veineuse chronique, l'oedème des membranes inférieurs et la maladie hémorroïdaire.

**Revendication pour les Etats contractants suivants: ES, GR**

1. Procédé de préparation des composés de formule I :

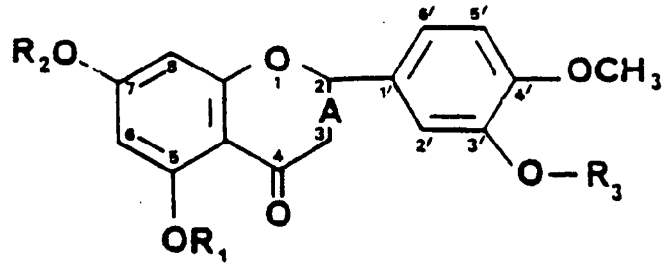

(I)

dans laquelle :

– A représente une simple ou une double liaison,

– $R_1$, $R_3$ identiques ou différents représentent chacun un atome d'hydrogène, un radical alcoxy carbonyl méthylène de formule $-CH_2CO_2R_4$ (dans laquelle $R_4$ représente un radical alkyle de 1 à 5 atomes de carbone) ou un radical de formule W :

W = 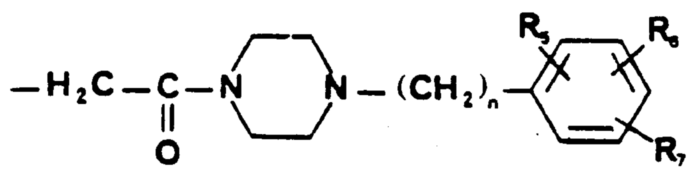

(dans laquelle n est égal à 0 ou 1 et $R_5$, $R_6$, $R_7$ identiques ou différents représentent chacun un atome d'hydrogène ou d'halogène, un radical hydroxyle, un radical trifluorométhyle, un radical alkyle inférieur renfermant de 1 à 5 atomes de carbone ou un radical alcoxy renfermant de 1 à 5 atomes de carbone),

– $R_2$ représente un atome d'hydrogène, un radical alcoxy carbonyl méthylène de formule $-CH_2CO_2R_4$, un radical de formule W, ou une molécule de β-glucose ou rutinose liée à l'oxygène auquel elle est attachée avec une liaison osidique,

à condition toutefois qu'au moins ou $R_1$, ou $R_2$, ou $R_3$ représente toujours un radical de formule W,

et leurs sels d'addition à un acide minéral ou organique pharmaceutiquement acceptable,

caractérisé en ce que :

soit :

l'on fait réagir la diosmine, composé de formule II :

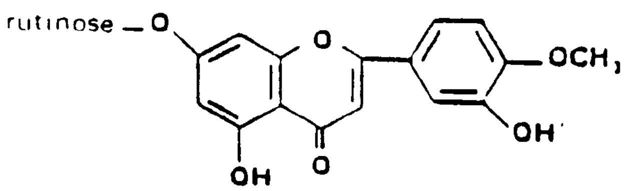

(II)

avec un chlorure d'alkyle de formule III renfermant les composés de formules générales IIIa et IIIb :

$ClCH_2CO_2R_4$ IIIa

$Cl\,W$ IIIb

(III)

dans lesquels $R_4$ et W ont les mêmes significations que pour la formule I, dans un solvant organique azoté polaire, en présence d'un sel minéral acide de métal alcalin à une température comprise entre 80°-120°C, pour obtenir

- <u>ou</u>

avec les composés de formule IIIa, les composés de formule générale IV :

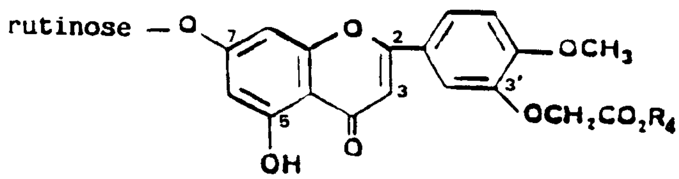

(IV)

dans laquelle $R_4$ a la signification indiquée pour la formule I,

que l'on soumet à une hydrolyse acide en présence d'un acide minéral concentré et à une température comprise entre 35°-55°C pour former le composé de formule V :

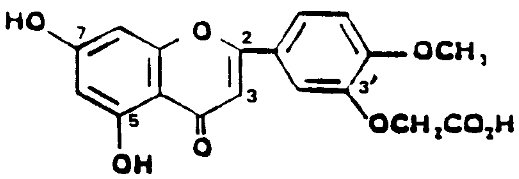

(V)

lequel ensuite :

- <u>soit :</u>

que l'on soumet à une acétylation à l'aide de l'anhydride acétique dans un solvant organique basique et à une température ambiante pour former le composé de formule VI :

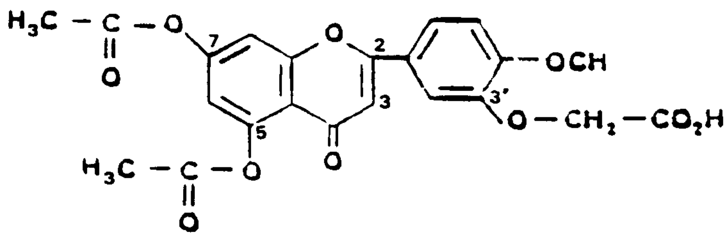

(VI)

que l'on fait réagir avec un dérivé de la pipérazine de formule générale VII :

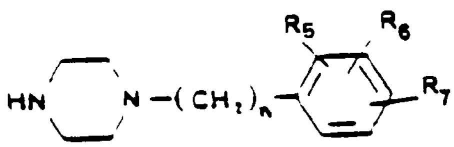

(VII)

dans laquelle n, $R_5$, $R_5$, $R_7$ ont la même signification que pour la formule I, en présence d'une amine tertiaire de petit poids moléculaire et de chloroformiate d'éthyle, à une température comprise entre 0°-20°C pour former les composés de formule générale VIII :

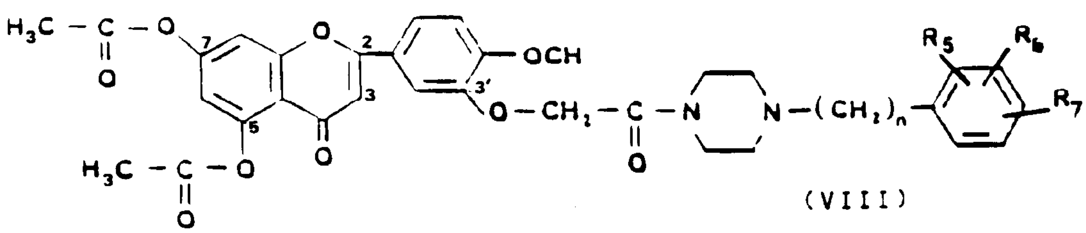

(VIII)

dans laquelle n, $R_5$, $R_6$, $R_7$ ont la signification indiquée, pour la formule I,

que l'on soumet ensuite à une désacétylation en solution dans le diméthylformamide en présence d'un sel minéral acide d'un métal alcalin à une température comprise entre 80°-120°C, pour former les composés de formule générale $I_A$ :

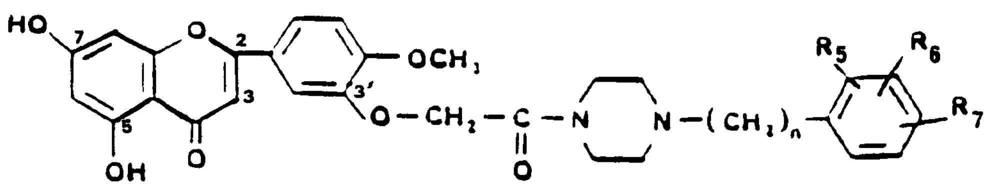

($I_A$)

dans laquelle la signification de n, $R_5$, $R_6$ et $R_7$ reste identique à celle donnée pour la formule I,

que l'on condense avec un composé de formule générale III pour former des composés de formule générale $I_B$ :

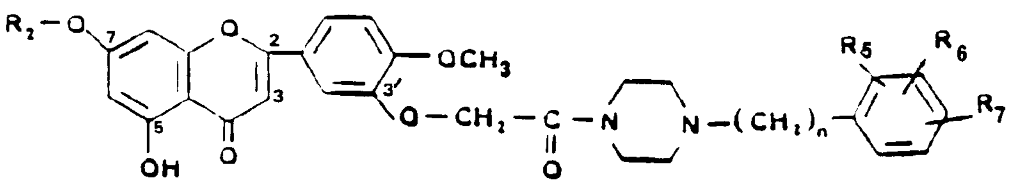

($I_B$)

dans laquelle n, $R_5$, $R_6$ et $R_7$ ont la signification indiquée ci-dessus et $R_2$ représente un radical alcoxy carbonyl méthylène de formule $-CH_2CO_2R_4$ ou un radical de formule W,

- soit :

que l'on estérifie avec un alcool primaire ou secondaire contenant de 1 à 5 atomes de carbone, en milieu anhydre à une température comprise entre 80°-110°C et en présence de l'acide tosylique pour former les composés de formule générale IX :

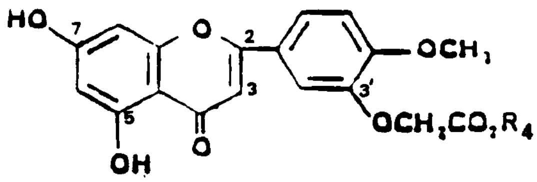

(IX)

dans laquelle $R_4$ a la même signification que pour la formule IV, que l'on fait réagir

■ ou avec un chlorure d'alkyle de formule générale $III_B$ pour former les composés de formule générale $I_C$ :

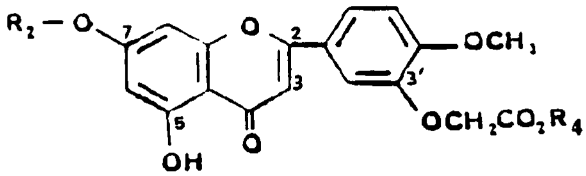

($I_C$)

dans laquelle $R_2$ représente un radical de formule W,

■ ou avec un chlorure d'alkyle de formule $III_A$ pour former les composés de formule générale X :

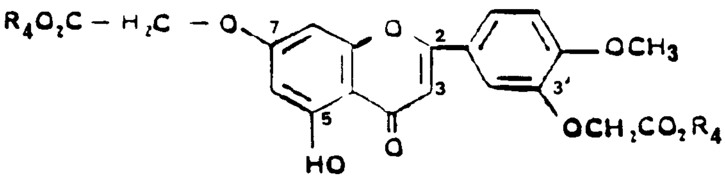

(X)

dans laquelle $R_4$ a la signification indiquée ci-dessus pour la formule I, que l'on condense avec un composé de formule générale $III_B$ pour obtenir les composés de formule générale $I_D$ :

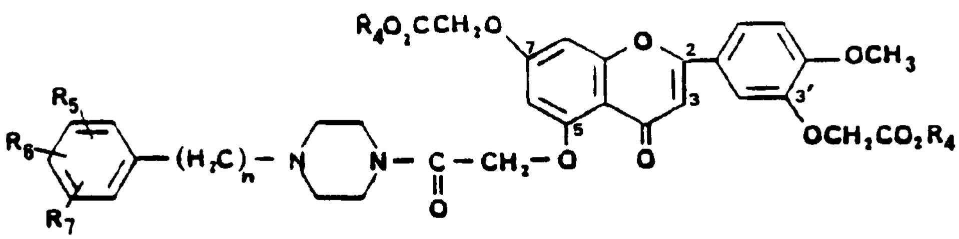

($I_D$)

dans laquelle la signification de n, $R_4$, $R_5$, $R_6$ et $R_7$ reste identique à celle donnée, pour la formule I,

- **ou**

avec les composés de formule $III_B$, les composés de formule générale $I_E$,

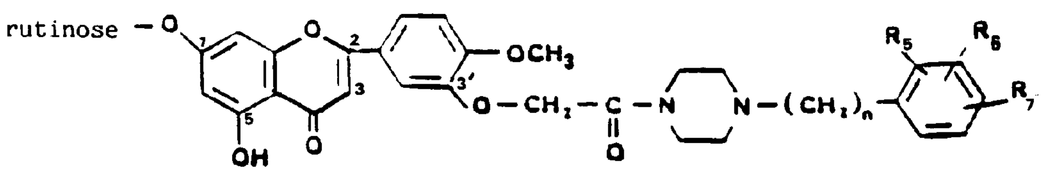

($I_E$)

dans laquelle n, $R_5$, $R_6$, $R_7$ ont la signification indiquée pour la formule I, que l'on soumet ensuite à une hydrolyse enzymatique à l'aide de naringinase pour former les composés

de formule générale $I_F$ :

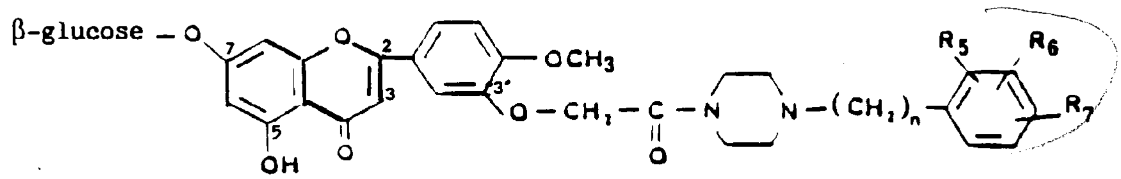

$(I_F)$

dans laquelle n, $R_5$, $R_6$ et $R_7$ ont la signification indiquée ci-dessus,

lesquels ensuite,

que l'on soumet à une hydrolyse enzymatique à l'aide de la β-glucosidase pour former les composés de formule générale $I_A$,

que l'on condense ensuite avec un composé de formule générale III pour former les composés de formule générale $I_B$,

soit :

l'on fait réagir un composé de formule générale XI :

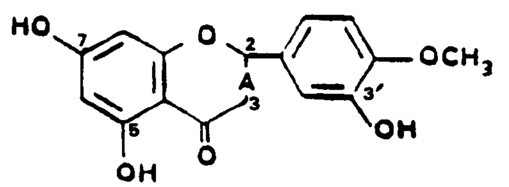

(XI)

dans laquelle A a la même signification que pour la formule I, avec une quantité adéquate des composés de formule générale $III_B$,

pour former les composés de formule générale $I_G$ :

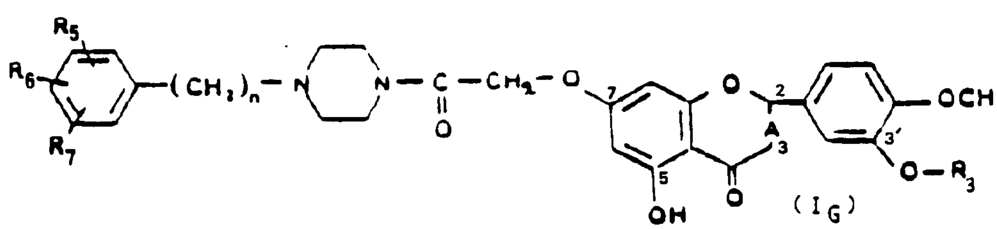

$(I_G)$

$(I_G)$

dans laquelle n, A, $R_5$, $R_6$ et $R_7$ ont la signification indiquée dans la formule I, et $R_3$ représente un atome d'hydrogène quand la quantité des composés de formule générale $III_B$ utilisée est environ équimolaire, ou un radical de formule W quand alkylation est effectuée avec une quantité de chlorure d'alkyle de formule générale $III_B$ au moins double,

et que l'on salifie ensuite, si l'on désire, les composés des formules $I_A$ à $I_G$, qui forment l'ensemble des composés de formule I, avec un acide organique ou minéral pharmaceutiquement acceptable.

## Patentansprüche

### Patentansprüche für folgende Vertragsstaaten: AT, BE, CH, DE, FR, GB, IT, LI, LU, NL, SE

1. Verbindungen der Formel 1

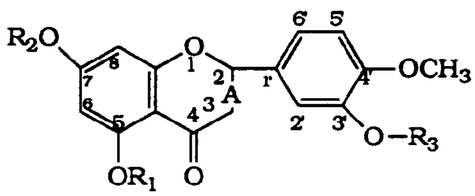

(I)

in der
– A eine Einfachbindung oder eine Doppelbindung,
– $R_1$ und $R_3$, die gleichartig oder verschieden sein können, jeweils Wasserstoffatome, Alkoxycarbonylmethylengruppen der Formel $-CH_2CO_2R_4$ (worin $R_4$ für eine Alkylgruppe mit 1 bis 5 Kohlenstoffatomen steht) oder eine Gruppe der Formel W

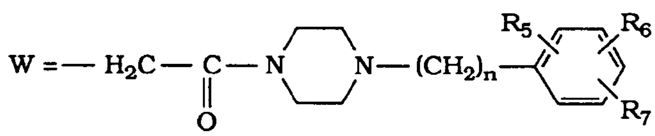

(worin n 0 oder 1 und $R_5$, $R_6$ und $R_7$, die gleichartig oder verschieden sein können, jeweils Wasserstoffatome, Halogenatome, Hydroxylgruppen, Trifluormethylgruppen, niedrigmolekulare Alkylgruppen mit 1 bis 5 Kohlenstoffatomen oder Alkoxygruppen mit 1 bis 5 Kohlenstoffatomen darstellen),
– $R_2$ ein Wasserstoffatom, eine Alkoxycarbonylmethylengruppe der Formel $-CH_2CO_2R_4$ eine Gruppe der Formel W oder ein an das Sauerstoffatom über eine oSidische Bindung gebundenes β-Glucose- oder Rutinose-Molekül
mit der Maßgabe bedeuten, daß mindestens eine der Gruppen $R_1$ oder $R_2$ oder $R_3$ stets eine Gruppe der Formel W darstellt,
und deren Additionssalze mit einer anorganischen oder organischen, pharmazeutisch annehmbaren Säure.

2. Das 7-Rhamnoglucosid von 3′-[2-{4-[(2,3,4-trimethoxy-phenyl)-methyl]-piperazin-1-yl}-2-oxo-ethoxy]-4′-methoxy-5-hydroxy-flavon als Verbindung der allgemeinen Formel I nach Anspruch 1, und dessen Additionssalze mit einer anorganischen oder organischen, pharmazeutisch annehmbaren Säure.

3. 3′-[2-{4-[(2,3,4-trimethoxy-phenyl)-methyl]-piperazin-1-yl}-2-oxo-ethoxy]-4′-methoxy-5,7-dihydroxy-flavon als Verbindung der Formel I von Anspruch 1 und dessen Additionssalze mit einer anorganischen oder organischen, pharmazeutisch annehmbaren Säure.

4. Verfahren zur Herstellung der Verbindungen der Formel I nach Anspruch 1, **dadurch gekennzeichnet,** daß man
**entweder:**
das Diosmin der Formel II

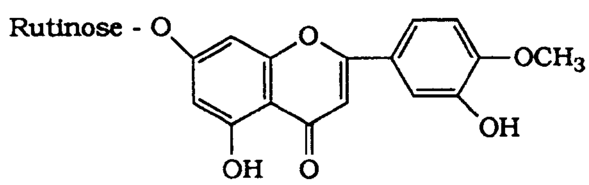

(II)

mit einem Alkylchlorid der Formel III, welche die Verbindungen der allgemeinen Formeln IIIa und IIIb umfaßt:

$$\left\{ \begin{array}{ll} ClCH_2CO_2R_4 & \text{IIIa} \\ ClW & \text{IIIb} \end{array} \right\} \text{III}$$

worin $R_4$ und W die bezüglich der Formel I in Anspruch 1 angegebenen Bedeutungen besitzen, in einem stickstoffhaltigen polaren organischen Lösungsmittel in Gegenwart eines Alkalimetallsalzes einer anorganischen Säure bei einer Temperatur zwischen 80 und 120°C umsetzt zur Bildung

**entweder**

mit den Verbindungen der Formel IIIa, von Verbindungen der allgemeinen Formel IV

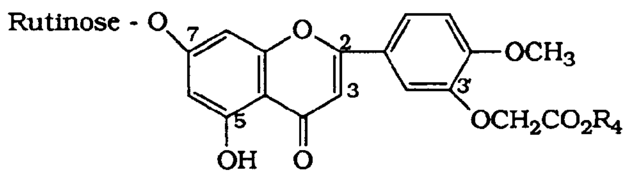

(IV)

in der $R_4$ die bezüglich der Formel I in Anspruch 1 angegebenen Bedeutungen besitzt,

welche man einer sauren Hydrolyse in Gegenwart einer konzentrierten anorganischen Säure bei einer Temperatur zwischen 35 und 55°C unterwirft zur Bildung der Verbindung der Formel V

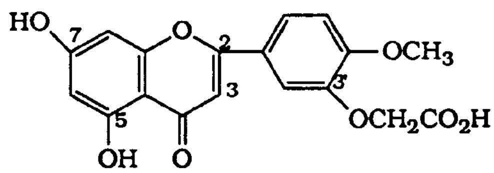

(V)

welche anschließend:

**entweder:**

mit Acetanhydrid in einem basischen organischen Lösungsmittel bei Raumtemperatur zur Bildung der Verbindung der Formel VI

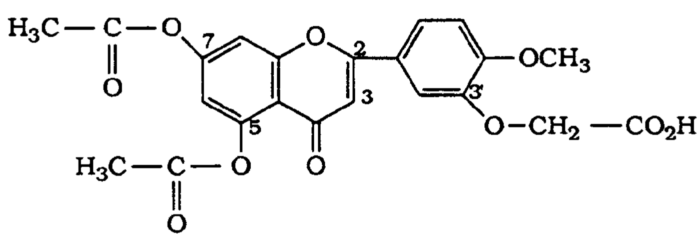

(VI)

acetyliert wird, welche man mit einem Piperazinderivat der allgemeinen Formel VII

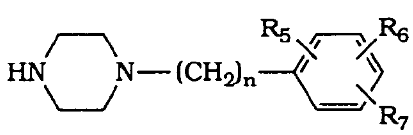

(VII)

in der n, $R_5$, $R_6$ und $R_7$ die bezüglich der Formel I in Anspruch 1 angegebenen Bedeutungen besitzen, in Gegenwart eines tertiären Amins mit niedrigem Molekulargewicht und Chlorameisensäureethylester bei einer Temperatur zwischen 0 und 20°C umsetzt zur Bildung der Verbindungen der allgemeinen Formel VIII

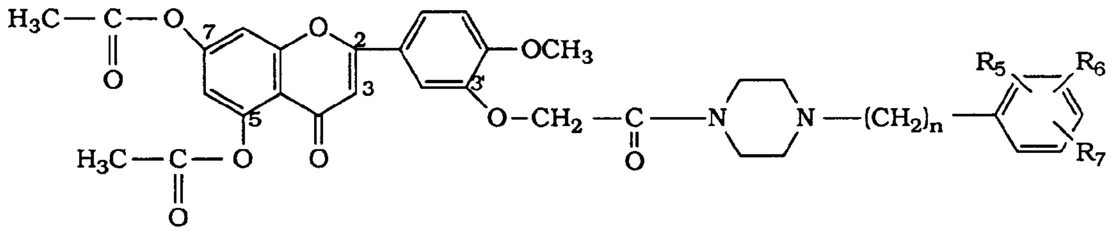

(VIII)

in der n, $R_5$, $R_6$ und $R_7$ die bezüglich der Formel I in Anspruch 1 angegebenen Bedeutungen besitzen,

welche man anschließend in Lösung in Dimethylformamid in Gegenwart eines Alkalimetallsalzes einer anorganischen Säure bei einer Temperatur zwischen 80 und 120°C desacetyliert zur Bildung der Verbindungen der allgemeinen Formel $I_A$

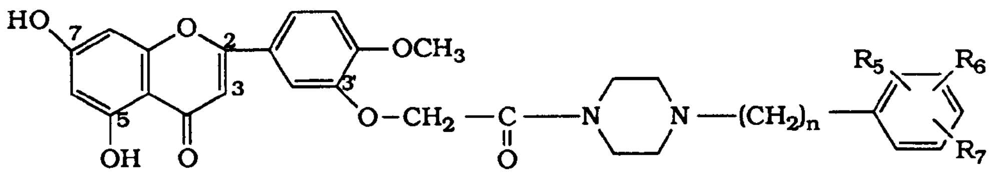

($I_A$)

in der die Bedeutungen von n, $R_5$, $R_5$ und $R_7$ gleich sind mit jenen, die bezüglich der Formel I in Anspruch 1 angegeben sind,

welche man mit einer Verbindung der allgmeinen Formel III kondensiert zur Bildung von Verbindungen der allgemeinen Formel $I_B$

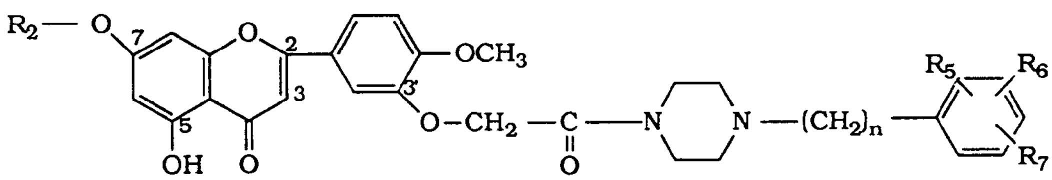

($I_B$)

in der n, $R_5$, $R_6$ und $R_7$ die oben angegebenen Bedeutungen besitzen und $R_2$ eine Alkoxycarbonylmethylengruppe der Formel - $CH_2CO_2R_4$ oder eine Gruppe der Formel W, wie sie in Anspruch 1 angegeben ist, bedeutet,

**oder:**

mit einem primären oder sekundären Alkohol mit 1 bis 5 Kohlenstoffatomen in wasserfreiem Medium in Gegenwart von Tosylsäure bei einer Temperatur zwischen 80 und 110°C verestert wird, zur Bildung der Verbindungen der allgemeinen Formel IX

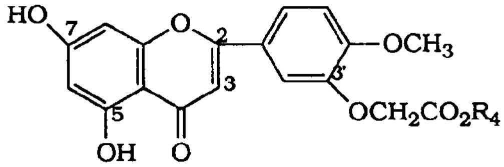

(IX)

worin $R_4$ die bezüglich der Formel IV angegebenen Bedeutungen besitzt, welche man

■ mit einem Alkylchlorid der allgemeinen Formel $III_B$ zur Bildung der Verbindungen der allgemeinen Formel $I_C$ umsetzt:

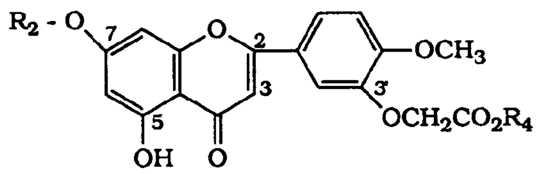

($I_C$)

in der $R_2$ eine Gruppe der Formel W, wie sie in Anspruch 1 definiert ist, bedeutet,

■ oder mit einem Alkylchlorid der Formel $III_A$ zur Bildung der Verbindungen der allgemeinen Formel X umsetzt:

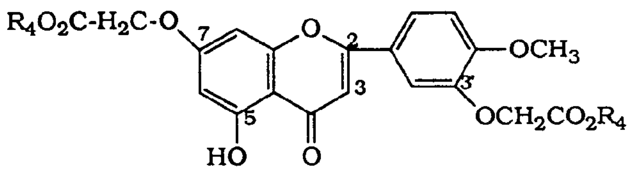

(X)

in der $R_4$ die oben bezüglich der Formel I in Anspruch 1 angegebenen Bedeutungen besitzt, welche man mit einer Verbindung der allgemeinen Formel $III_B$ kondensiert zur Bildung der Verbindungen der allgemeinen Formel $I_D$

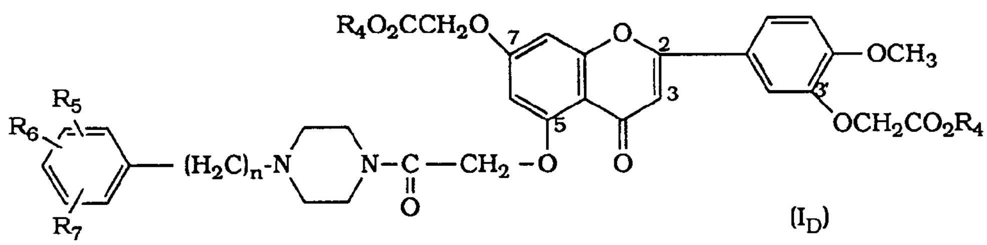

($I_D$)

in der die Bedeutungen von n, $R_4$, $R_5$, $R_6$ und $R_7$ gleich sind jenen, die in Anspruch 1 bezüglich der Formel I angegeben sind,

**- oder**

mit den Verbindungen der Formel $III_B$ zu den Verbindungen der allgemeinen Formel $I_E$ umsetzt:

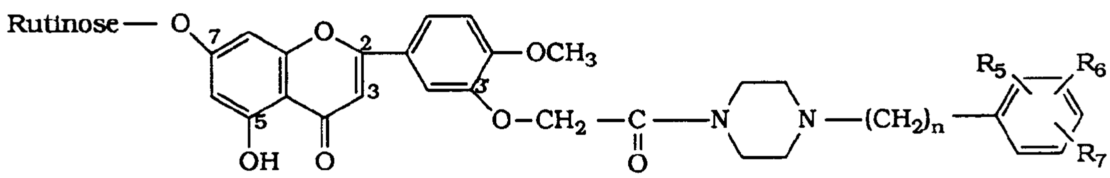

($I_E$)

worin n, $R_5$, $R_6$ und $R_7$ die bezüglich der Formel I in Anspruch 1 angegebenen Bedeutungen besitzen, welche man anschließend enzymatisch mit Hilfe von Naringinase hydrolysiert zur Bildung der Verbindungen der allgemeinen Formel $I_F$

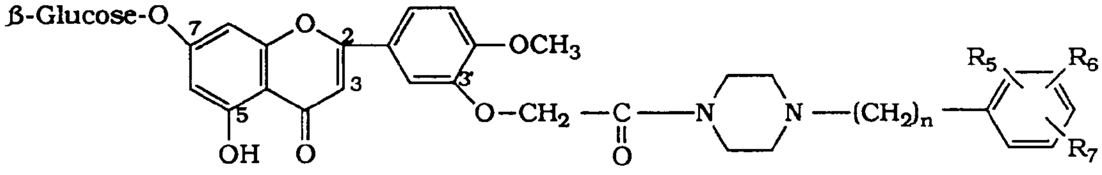

$(I_F)$

in der n, $R_5$, $R_6$ und $R_7$ die oben angegebenen Bedeuten besitzen,

welche man anschließend einer enzymatischen Hydrolyse mit β-Glucosidase unterwirft zur Bildung der Verbindungen der allgemeinen Formel $I_A$,

welche man anschließend mit einer Verbindung der allgemeinen Formel III kondensiert zur Bildung der Verbindungen der allgemeinen Formel $I_B$,

**oder**:

eine Verbindung der allgemeinen Formel XI

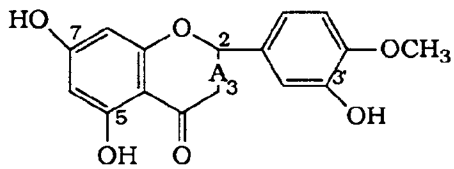

(XI)

in der A die bezüglich der Formel I in Anspruch 1 angegebenen Bedeutungen besitzt, mit einer geeigneten Menge der Verbindung der allgemeinen Formel $III_B$ umsetzt,

zur Bildung der Verbindungen der allgemeinen Formel $I_G$

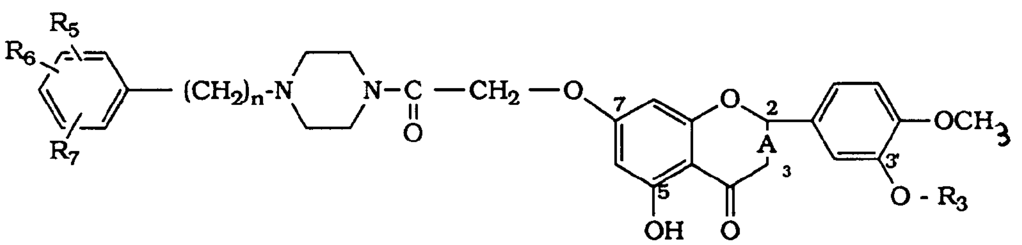

$(I_G)$

in der n, A, $R_5$, $R_5$ und $R_7$ die in Anspruch 1 bezüglich der Formel I angegebenen Bedeutungen besitzen und $R_3$ ein Wasserstoffatom darstellt, wenn die verwendete Menge der Verbindung der allgemeinen Formel $III_B$ etwa äquimolar ist, oder eine Gruppe der in Anspruch 1 definierten Formel W bedeutet, wenn die Alkylierung mit einer mindestens doppelten Menge des Alkylchlorids der allgemeinen Formel $III_B$ durchgeführt worden ist,

und gewünschtenfalls die Verbindungen der Formel $I_A$ bis $I_G$, welche die Gesamtheit der Verbindungen der Formel I gemäß Anspruch 1 darstellen, mit einer organischen oder anorganischen, pharmazeutisch annehmbaren Säure in ein Salz überführt.

5. Pharmazeutische Zubereitung, enthaltend als Wirkstoff eine Verbindung nach einem der Ansprüche 1 bis 3 in Kombination oder in Mischung mit einem inerten, nichttoxischen, pharmazeutisch annehmbaren Trägermaterial und/oder Bindemittel.

6. Pharmazeutische Zubereitung nach Anspruch 5, enthaltend den Wirkstoff in einer Dosis von 50 bis 500 mg.

7. Pharmazeutische Zubereitung gemaß den Ansprüchen 5 und 6, enthaltend als Wirkstoff mindestens eine Verbindung nach einem der Ansprüche 1 bis 3 zur Verwendung bei der Behandlung von Gefäßerkrankungen, wie chronische Veneninsuffizienz, Ödeme der unteren Gliedmaßen und Hämorrhoidenerkrankungen.

**Patentansprüche für folgende Vertragsstaaten: ES, GR**

1. Verfahren zur Herstellung der Verbindungen der allgemeinen Formel I

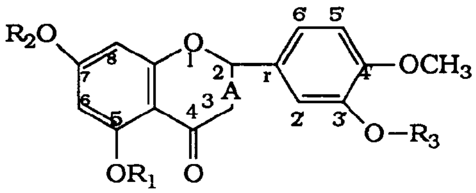

(I)

in der

– A eine Einfachbindung oder eine Doppelbindung,

– $R_1$ und $R_3$, die gleichartig oder verschieden sein können, jeweils Wasserstoffatome, Alkoxycarbonylmethylengruppen der Formel $-CH_2CO_2R_4$ (worin $R_4$ für eine Alkylgruppe mit 1 bis 5 Kohlenstoffatomen steht) oder eine Gruppe der Formel W

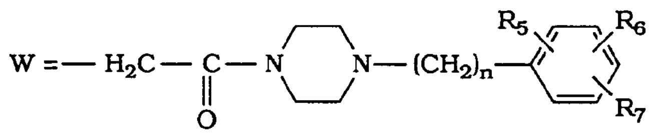

(worin n 0 oder 1 und $R_5$, $R_6$ und $R_7$, die gleichartig oderverschieden sein können, jeweils Wasserstoffatome, Halogenatome, Hydroxylgruppen, Trifluormethylgruppen, niedrigmolekulare Alkylgruppen mit 1 bis 5 Kohlenstoffatomen oder Alkoxygruppen mit 1 bis 5 Kohlenstoffatomen darstellen),

– $R_2$ ein Wasserstoffatom, eine Alkoxycarbonylmethylengruppe der Formel $-CH_2CO_2R_4$ eine Gruppe der Formel W oder ein an das Sauerstoffatom über eine osidische Bindung gebundenes β-Glucose- oder Rutinose-Molekül mit der Maßgabe bedeuten, daß mindestens eine der Gruppen $R_1$ oder $R_2$ oder $R_3$ stets eine Gruppe der Formel W darstellt,

und deren Additionssalzen mit einer anorganischen oder organischen, pharmazeutisch annehmbaren Saure,

**dadurch gekennzeichnet,** daß man

**entweder**:

Diosmin der Formel II

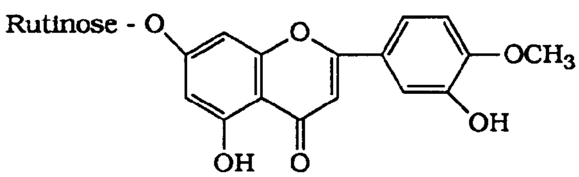

(II)

mit einem Alkylchlorid der Formel III, welche die Verbindungen der allgemeinen Formeln IIIa und IIIb umfaßt:

$$\left\{ \begin{array}{ll} ClCH_2CO_2R_4 & \mathrm{IIIa} \\ ClW & \mathrm{IIIb} \end{array} \right\} \quad \mathrm{III}$$

worin $R_4$ und W die bezüglich der Formel I in Anspruch 1 angegebenen Bedeutungen besitzen, in einem stickstoffhaltigen polaren organischen Lösungsmittel in Gegenwart eines Alkalimetallsalzes einer anorganischen Säure bei einer Temperatur zwischen 80 und 120°C umsetzt zur Bildung

**entweder**

mit den Verbindungen der Formel IIIa, von Verbindungen der allgemeinen Formel IV

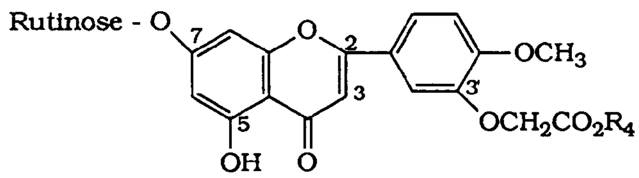

(IV)

in der $R_4$ die bezüglich der Formel I angegebenen Bedeutungen besitzt, welche man einer sauren Hydrolyse in Gegenwart einer konzentrierten anorganischen Säure bei einer Temperatur zwischen 35 und 55°C unterwirft zur Bildung der Verbindung der Formel V

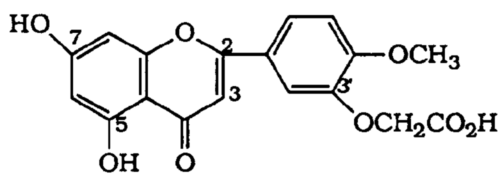

(V)

welche anschließend:

**entweder:**

mit Acetanhydrid in einem basischen organischen Lösungsmittel bei Raumtemperatur zur Bildung der Verbindung der Formel VI

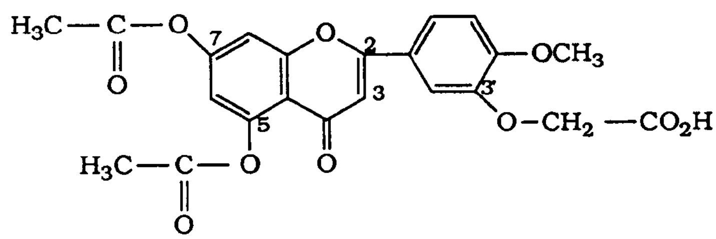

(VI)

acetyliert wird, welche man mit einem Piperazinderivat der allgemeinen Formel VII

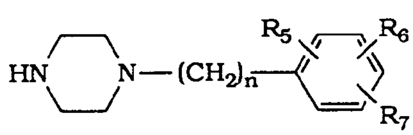

(VII)

in der n, $R_5$, $R_6$ und $R_7$ die bezüglich der Formel I angegebenen Bedeutungen besitzen, in Gegenwart eines tertiären Amins mit niedrigem Molekulargewicht und Chlorameisensäureethylester bei einer Temperatur zwischen 0 und 20°C umsetzt zur Bildung der Verbindungen der allgemeinen Formel VIII

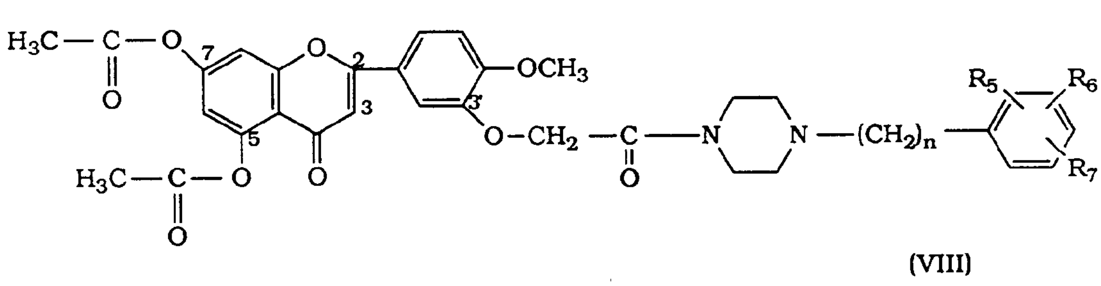

(VIII)

in der n, $R_5$, $R_6$ und $R_7$ die bezüglich der Formel I angegebenen Bedeutungen besitzen,

welche man anschließend in Lösung in Dimethylformamid in Gegenwart eines Alkalimetallsalzes einer anorganischen Säure bei einer Temperatur zwischen 80 und 120°C desacetyliert zur Bildung der Verbindungen der allgemeinen Formel $I_A$

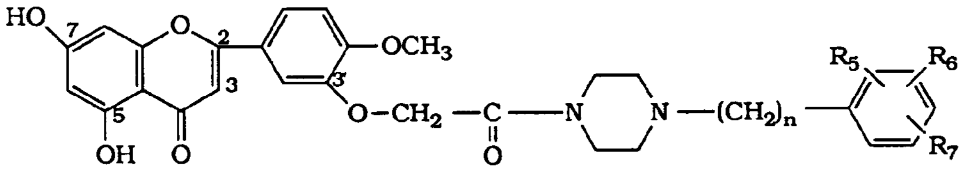

($I_A$)

in der die Bedeutungen von n, $R_5$, $R_5$ und $R_7$ gleich sind mit jenen, die bezüglich der Formel I angegeben sind,

welche man mit einer Verbindung der allgemeinen Formel III kondensiert zur Bildung von Verbindungen der allgemeinen Formel $I_B$

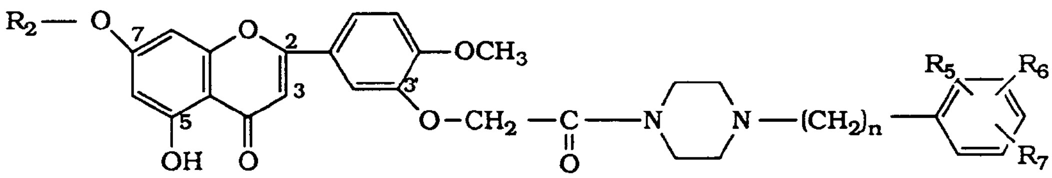

($I_B$)

in der n, $R_5$, $R_6$ und $R_7$ die oben angegebenen Bedeutungen besitzen und $R_2$ eine Alkoxycarbonylmethylengruppe der Formel $-CH_2CO_2R_4$ oder eine Gruppe der Formel W bedeutet,

**oder:**

die mit einem primären oder sekundären Alkohol mit 1 bis 5 Kohlenstoffatomen in wasserfreiem Medium in Gegenwart von Tosylsäure bei einer Temperatur zwischen 80 und 110°C verestert wird zur Bildung der Verbindungen der allgemeinen Formel IX

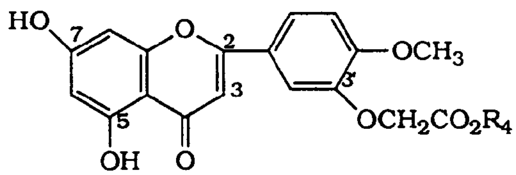

(IX)

worin $R_4$ die bezüglich der Formel IV angegebenen Bedeutungen besitzt, welche man

■ mit einem Alkylchlorid der allgemeinen Formel $III_B$ zur Bildung der Verbindungen der allgemeinen Formel $I_C$ umsetzt:

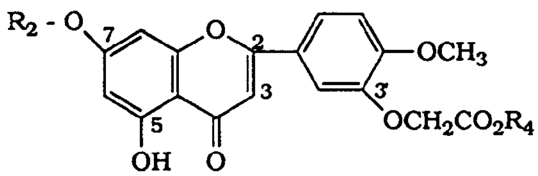

($I_C$)

in der $R_2$ eine Gruppe der Formel W bedeutet,

■ oder mit einem Alkylchlorid der Formel $III_A$ zur Bildung der Verbindungen der allgemeinen Formel X umsetzt:

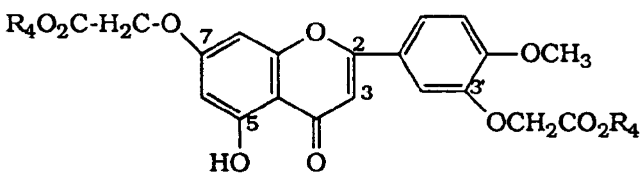

(X)

in der $R_4$ die oben bezüglich der Formel I angegebenen Bedeutungen besitzt, welche man mit einer Verbindung der allgemeinen Formel $III_B$ kondensiert zur Bildung der Verbindungen der allgemeinen Formel $I_D$

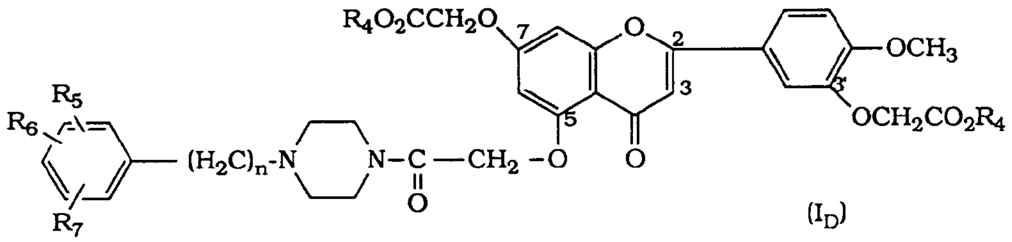

$(I_D)$

in der die Bedeutungen von n, $R_4$, $R_5$, $R_6$ und $R_7$ gleich sind jenen, die bezüglich der Formel I angegeben sind,

**- oder**

mit den Verbindungen der Formel $III_B$ zu den Verbindungen der allgemeinen Formel $I_E$ umsetzt:

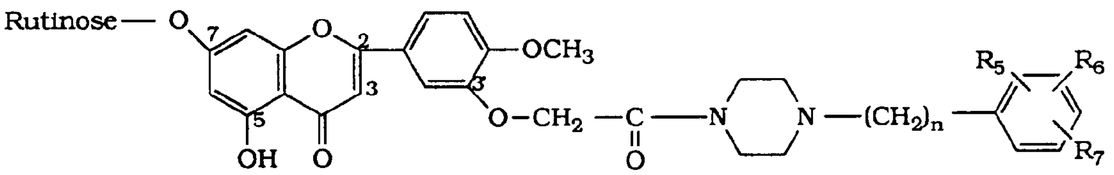

$(I_E)$

worin n, $R_5$, $R_6$ und $R_7$ die bezüglich der Formel I angegebenen Bedeutungen besitzen,

welche man anschließend enzymatisch mit Hilfe von Naringinase hydrolysiert zur Bildung der Verbindungen der allgemeinen Formel $I_F$

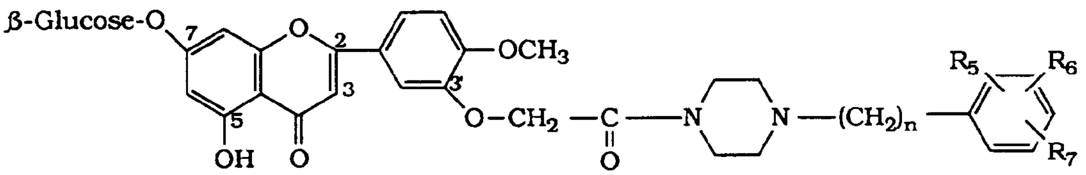

$(I_F)$

in der n, $R_6$, $R_6$ und $R_7$ die oben angegebenen Bedeuten besitzen,

welche man anschließend einer enzymatischen Hydrolyse mit β-Glucosidase unterwirft zur Bildung der Verbindungen der allgemeinen Formel $I_A$,

welche man anschließend mit einer Verbindung der allgemeinen Formel III kondensiert zur Bildung der Verbindungen der allgemeinen Formel $I_B$,

**oder:**

eine Verbindung der allgemeinen Formel XI

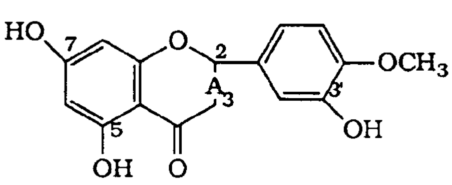

(XI)

in der A die bezüglich der Formel I angegebenen Bedeutungen besitzt, mit einer geeigneten Menge der Verbindung der allgemeinen Formel $III_B$ umsetzt zur Bildung der Verbindungen der allgemeinen Formel $I_G$

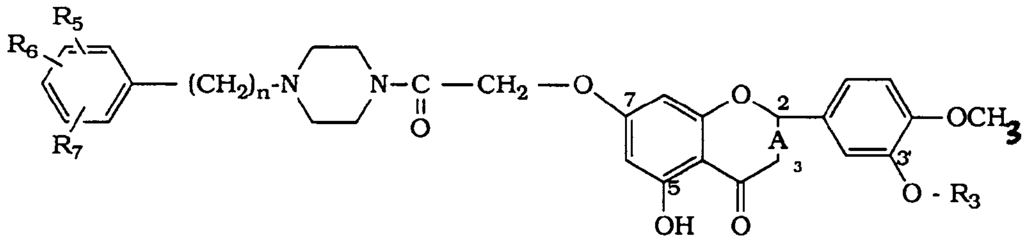

$(I_G)$

in der n, A, $R_5$, $R_6$ und $R_7$ die bezüglich der Formel I angegebenen Bedeutungen besitzen und $R_3$ ein Wasserstoffatom darstellt, wenn die verwendete Menge der Verbindung der allgemeinen Formel $III_B$ etwa äquimolar ist, oder eine Gruppe der Formel W bedeutet, wenn die Alkylierung mit einer mindestens doppelten Menge des Alkylchlorids der allgemeinen Formel $III_B$ durchgeführt worden ist,

und gewünschtenfalls die Verbindungen der Formel $I_A$ bis $I_G$, welche die Gesamtheit der Verbindungen der Formel I darstellen, mit einer organischen oder anorganischen, pharmazeutisch annehmbaren Säure in ein Salz überführt.

## Claims

### Claims for the following Contracting States: AT, BE, CH, DE, FR, GB, IT, LI, LU, NL, SE

1. Compounds of formula I :

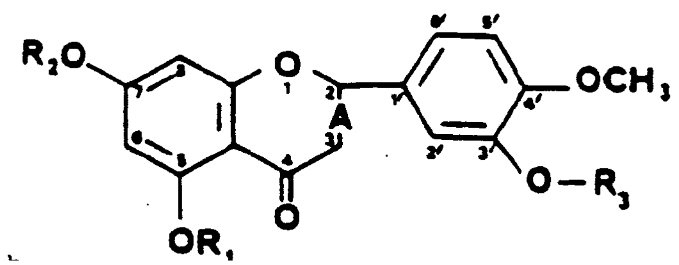

(I)

in which :

– A represents a single or double bond,

– $R_1$ and $R_3$, which may be the same or different, each represents a hydrogen atom, an alkoxycarbonylmethylene radical of the formula $-CH_2CO_2R_4$ (in which $R_4$ represents an alkyl radical having from 1 to 5 carbon atoms) or a radical of the formula W :

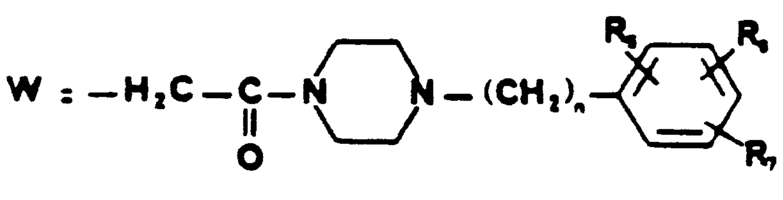

(in which n is equal to 0 or 1 and $R_5$, $R_6$ and $R_7$, which may be the same or different, each represents a hydrogen or halogen atom, a hydroxy radical, a trifluoromethyl radical, a lower alkyl radical containing from 1 to 5 carbon atoms or an alkoxy radical containing from 1 to 5 carbon atoms),

– $R_2$ represents a hydrogen atom, an alkoxycarbonylmethylene radical of the formula $-CH_2CO_2R_4$, a radical of the formula W, or a β-glucose or rutinose molecule bonded to the oxygen to which it is attached by a glycoside bond,

with the proviso, however, that at least $R_1$, $R_2$ or $R_3$ always represents a radical of the formula W,

and their addition salts with a pharmaceutically acceptable organic or mineral acid.

2. The 7-rhamnoglucoside of 3′-[2-{4-(2,3,4-trimethoxyphenylmethyl)-1-piperazinyl}-2-oxoethoxy]-4′-methoxy-5-hydroxyflavone, which compound corresponds to formula I according to claim 1, and its addition salts with a pharmaceutically acceptable organic or mineral acid.

3. 3'-[2-{4-(2,3,4-trimethoxyphenylmethyl)-1-piperazinyl}-2-oxoethoxy]-4'-methoxy-5,7-dihydroxyflavone, which compound corresponds to formula I according to claim 1, and its addition salts with a pharmaceutically acceptable organic or mineral acid.

4. Process for the preparation of compounds of formula I according to claim 1, characterised in that :

either :

diosmin, a compound of formula II :

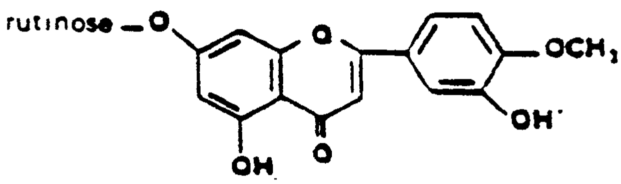

(II)

is reacted with an alkyl chloride of formula III, which comprises compounds of the general formulae IIIa and IIIb :

$ClCH_2CO_2R_4$ IIIa

Cl W IIIb

(III)

in which $R_4$ and W are as defined for formula I according to claim 1, in a polar organic nitrogenous solvent, in the presence of an alkali metal salt of a mineral acid, at a temperature of between 80° and 120°C,

to obtain

- either

with compounds of formula IIIa, compounds of the general formula IV :

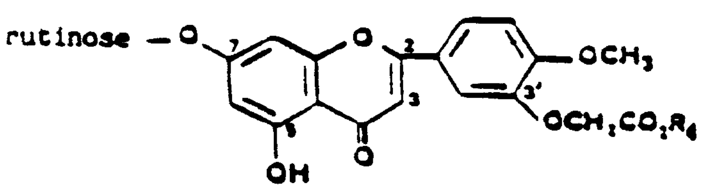

(IV)

in which $R_4$ is as defined for formula I according to claim 1,

which are subjected to acid hydrolysis in the presence of a concentrated mineral acid at a temperature of between 35° and 55°C, to form a compound of formula V :

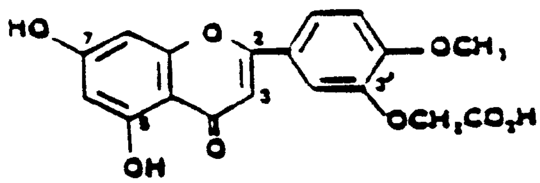

(V)

which is then :

-either :

subjected to acetylation using acetic anhydride in a basic organic solvent at room temperature to yield a compound of formula VI :

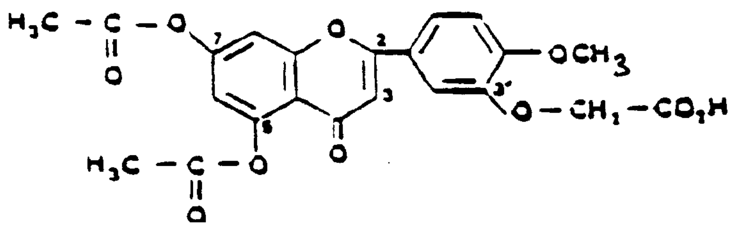

(VI)

which is reacted at a temperature of between 0° and 20°C, in the presence of a low molecular weight tertiary amine and ethyl chloroformate, with a piperazine compound of the general formula VII :

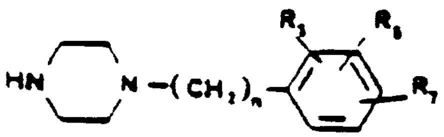

(VII)

in which n, $R_5$, $R_5$ and $R_7$ are as defined for formula I according to claim 1, to form compounds of the general formula VIII :

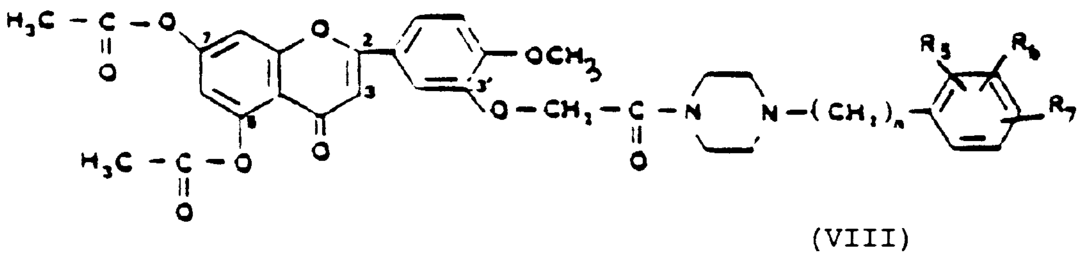

(VIII)

in which n, $R_5$, $R_5$ and $R_7$ are as defined above for formula I according to claim 1,

which are then subjected to deacetylation in solution in dimethylformamide in the presence of an alkali metal salt of a mineral acid at a temperature of between 80° and 120°C to yield compounds of the general formula $I_A$ :

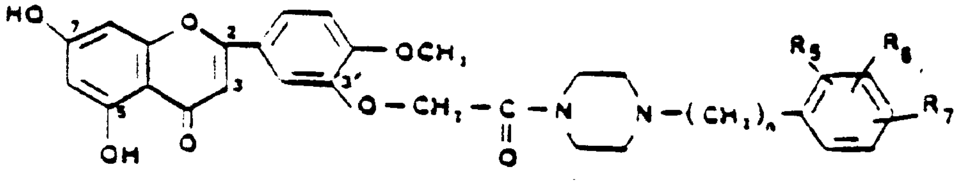

($I_A$)

in which the meanings of n, $R_5$, $R_6$ and $R_7$ are identical to those given for formula I according to claim 1,

which are condensed with a compound of the general formula III to form compounds of the general formula $I_B$:

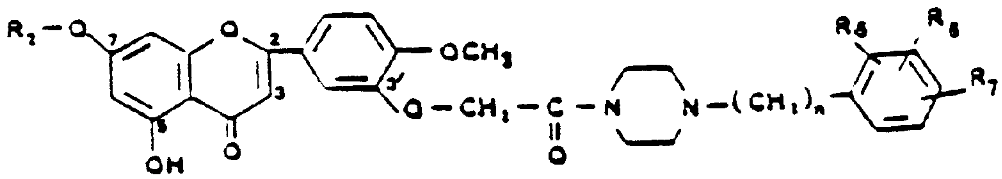

$(I_B)$

in which n, $R_5$, $R_5$ and $R_7$ are as defined above and $R_2$ represents an alkoxycarbonylmethylene radical of the formula $-CH_2CO_2R_4$ or a radical of the formula W according to claim 1,

- or :

is esterified at a temperature of between 80° and 110°C with a primary or secondary alcohol containing from 1 to 5 carbon atoms in anhydrous medium and in the presence of p-toluenesulphonic acid to form compounds of the general formula IX :

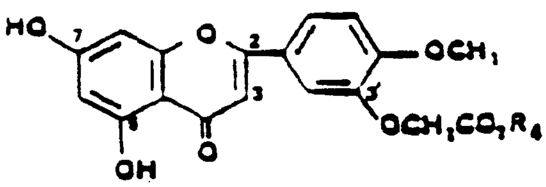

(IX)

in which $R_4$ is as defined for formula IV,

which are reacted

■ either with an alkyl chloride of the general formula $III_B$ to form compounds of the general formula $I_C$ :

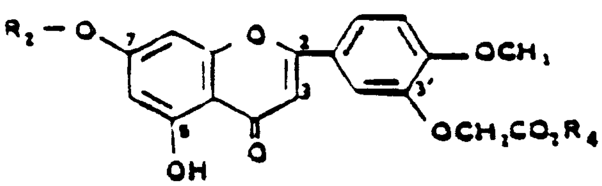

$(I_C)$

in which $R_2$ represents a radical of the formula W according to claim 1,

■ or with an alkyl chloride of the formula $III_A$ to form compounds of the general formula X :

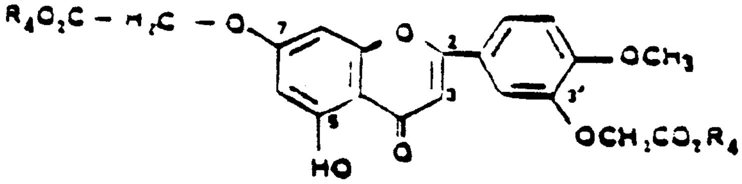

(X)

in which $R_4$ is as defined above for formula I according to claim 1,

which are condensed with a compound of the general formula $III_B$ to yield compounds of the general formula $I_D$ :

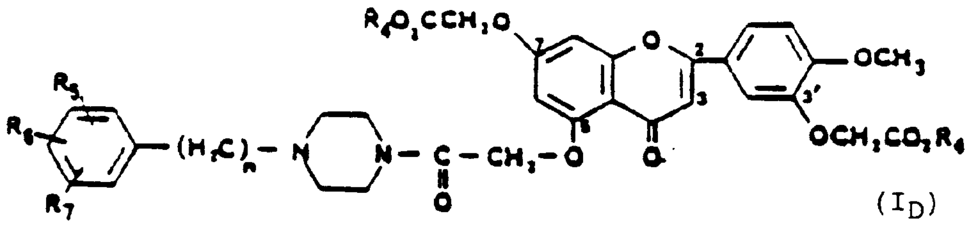

$(I_D)$

in which the meanings of n, $R_4$, $R_5$, $R_6$ and $R_7$ are identical to those given for formula I according to claim 1,

- or

with compounds of formula $III_B$, compounds of the general formula $I_E$

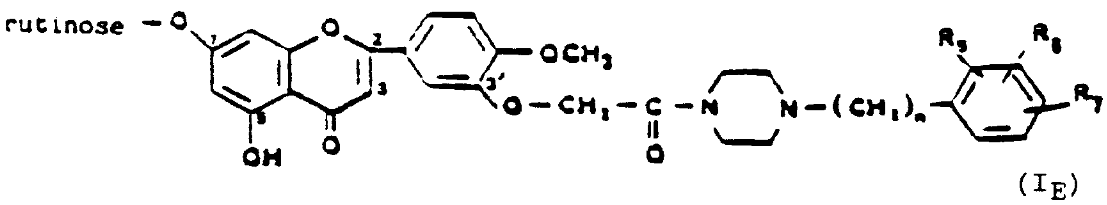

$(I_E)$

in which n, $R_5$, $R_5$ and $R_7$ are as defined for formula I according to claim 1,

which are then subjected to enzymatic hydrolysis using naringinase to yield compounds of the general formula $I_F$ :

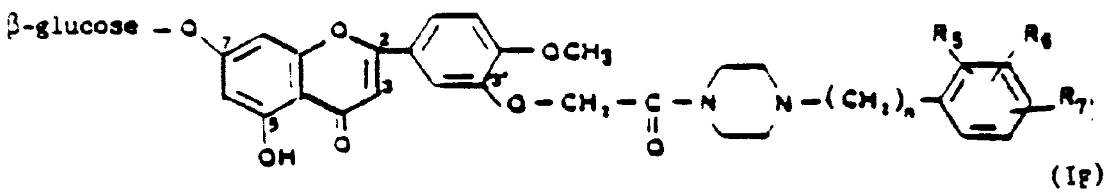

$(I_F)$

in which n, $R_5$, $R_5$ and $R_7$ are as defined above,

which are then

subjected to enzymatic hydrolysis using β-glucosidase to yield compounds of the general formula $I_A$,

which are then condensed with a compound of the general formula III to yield compounds of the general formula $I_B$,

or :

a compound of the general formula XI :

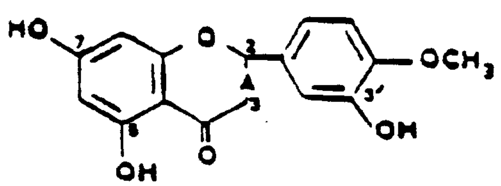

(XI)

in which A is as defined for formula I according to claim 1, is reacted with an appropriate amount of compounds of the general formula $III_B$,

to yield compounds of the general formula $I_G$ :

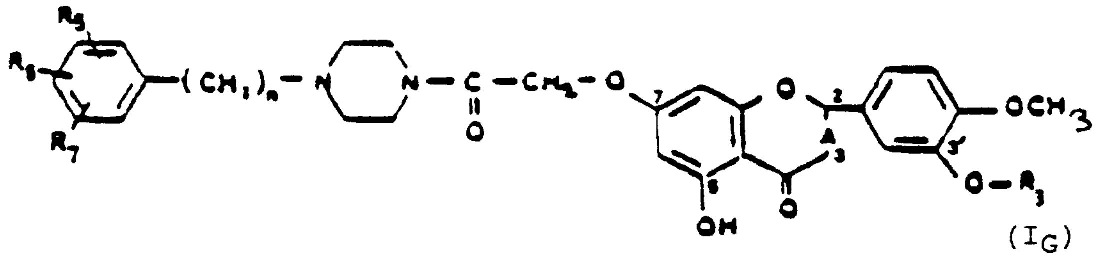

($I_G$)

in which n, A, $R_5$, $R_6$ and $R_7$ are as defined for formula I according to claim 1, and $R_3$ represents a hydrogen atom when the quantity of compounds of the general formula $III_B$ used is approximately equimolar, or a radical of the formula W according to claim 1 when the alkylation is carried out with at least double the amount of alkyl chloride of the general formula $III_B$,

and, if desired, the compounds of formulae $I_A$ to $I_G$, which form the totality of the compounds of formula I according to claim 1, are then converted into salts with a pharmaceutically acceptable mineral or organic acid.

5. Pharmaceutical composition containing as active ingredient a compound according to any one of claims 1 to 3, in association or in admixture with a pharmaceutically acceptable, non-toxic, inert vehicle or excipient.

6. Pharmaceutical composition according to claim 5 containing the active ingredient in an amount of from 50 to 500 mg.

7. Pharmaceutical composition according to claims 5 and 6 containing as active ingredient at least one compound according to claims 1 to 3 which can be used for the treatment of vascular disorders such as chronic venous insufficiency, oedema of the lower limbs and haemorrhoid disorders.

**Claim for the following Contracting States: ES, GR**

1. Process for the preparation of compounds of formula I :

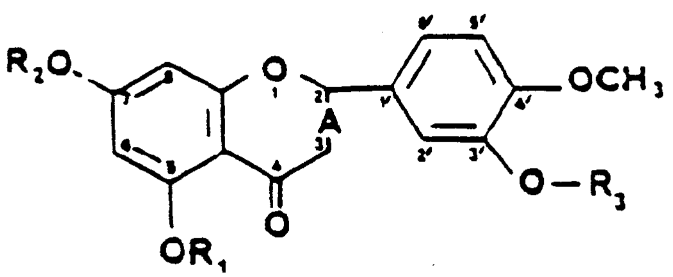

(I)

in which :

– A represents a single or double bond,

– $R_1$ and $R_3$, which may be the same or different, each represents a hydrogen atom, an alkoxycarbonylmethylene radical of the formula $-CH_2CO_2R_4$ (in which $R_4$ represents an alkyl radical having from 1 to 5 carbon atoms) or a radical of the formula W :

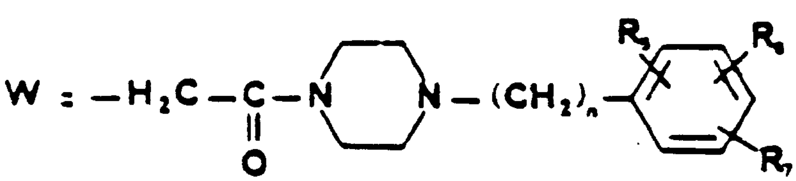

(in which n is equal to 0 or 1 and $R_5$, $R_5$ and $R_7$, which may be the same or different, each represents a hydrogen or halogen atom, a hydroxy radical, a trifluoromethyl radical, a lower alkyl radical containing from 1 to 5 carbon atoms or an alkoxy radical containing from 1 to 5 carbon atoms),

– $R_2$ represents a hydrogen atom, an alkoxycarbonylmethylene radical of the formula $-CH_2CO_2R_4$, a radical

of the formula W, or a β-glucose or rutinose molecule bonded to the oxygen to which it is attached by a glycoside bond,

with the proviso, however, that at least $R_1$, $R_2$ or $R_3$ always represents a radical of the formula W,

and their addition salts with a pharmaceutically acceptable organic or mineral acid,

characterised in that :

<u>either</u> :

diosmin, a compound of formula II :

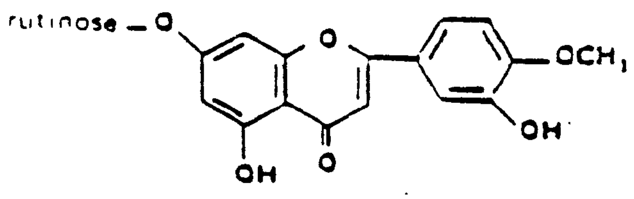

(II)

is reacted with an alkyl chloride of formula III, which comprises compounds of the general formulae IIIa and IIIb :

$ClCH_2CO_2R_4$ IIIa

Cl W IIIb

(III)

in which $R_4$ and W are as defined for formula I, in a polar organic nitrogenous solvent, in the presence of an alkali metal salt of a mineral acid, at a temperature of between 80° and 120°C, to obtain

- <u>either</u>

with compounds of formula IIIa, compounds of the general formula IV :

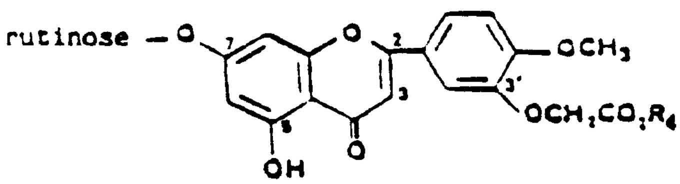

(IV)

in which $R_4$ is as defined for formula I,

which are subjected to acid hydrolysis in the presence of a concentrated mineral acid at a temperature of between 35° and 55°C, to form a compound of formula V :

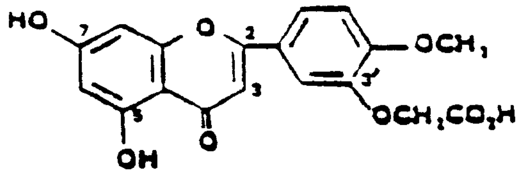

(V)

which is then :

-either :

subjected to acetylation using acetic anhydride in a basic organic solvent at room temperature to yield a compound of formula VI :

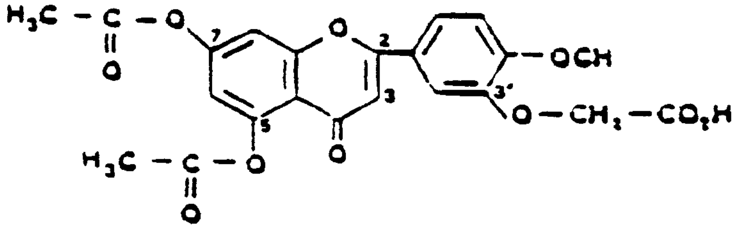

(VI)

which is reacted at a temperature of between 0° and 20°C, in the presence of a low molecular weight tertiary amine and ethyl chloroformate, with a piperazine compound of the general formula VII :

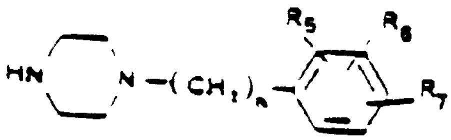

(VII)

in which n, $R_5$, $R_5$ and $R_7$ are as defined for formula I, to form compounds of the general formula VIII :

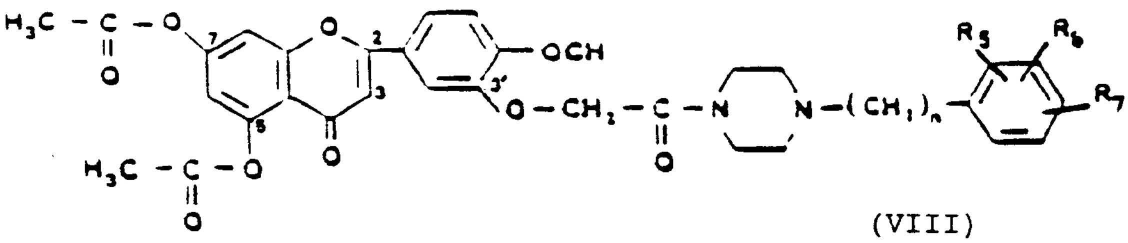

(VIII)

in which n, $R_5$, $R_5$ and $R_7$ are as defined above for formula I,

which are then subjected to deacetylation in solution in dimethylformamide in the presence of an alkali metal salt of a mineral acid at a temperature of between 80° and 120°C to yield compounds of the general formula $I_A$ :

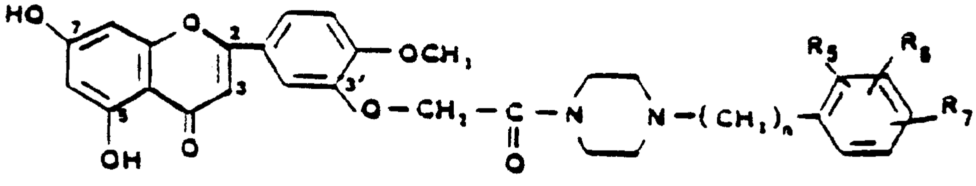

($I_A$)

in which the meanings of n, $R_5$, $R_6$ and $R_7$ are identical to those given for formula I,

which are condensed with a compound of the general formula III to form compounds of the general formula

$I_B$:

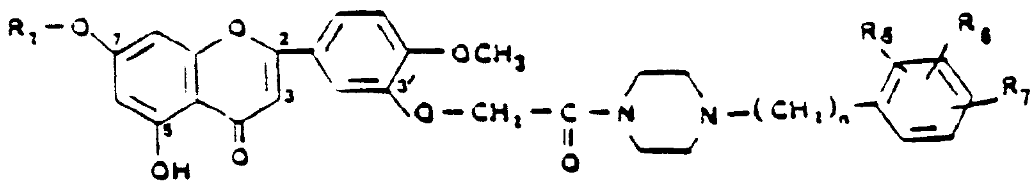

($I_B$)

in which n, $R_5$, $R_5$ and $R_7$ are as defined above and $R_2$ represents an alkoxycarbonylmethylene radical of the formula $-CH_2CO_2R_4$ or a radical of the formula W,

- or :

is esterified at a temperature of between 80° and 110°C with a primary or secondary alcohol containing from 1 to 5 carbon atoms in anhydrous medium in the presence of p-toluenesulphonic acid to form compounds of the general formula IX :

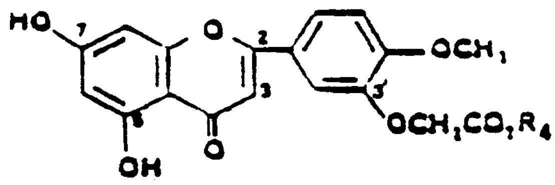

(IX)

in which $R_4$ is as defined for formula IV,

which are reacted

■ either with an alkyl chloride of the general formula $III_B$ to form compounds of the general formula $I_C$ :

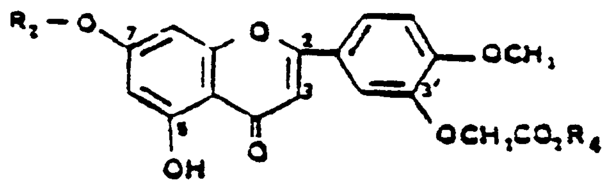

($I_C$)

in which $R_2$ represents a radical of the formula W,

■ or with an alkyl chloride of the formula $III_A$ to form compounds of the general formula X :

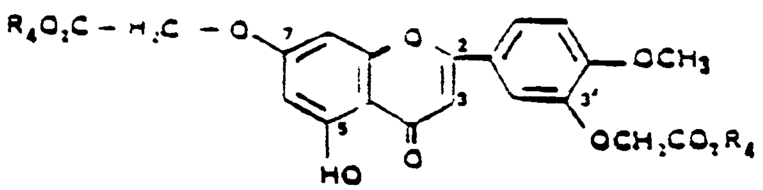

(X)

in which $R_4$ is as defined above for formula I,

which are condensed with a compound of the general formula $III_B$ to yield compounds of the general formula $I_D$ :

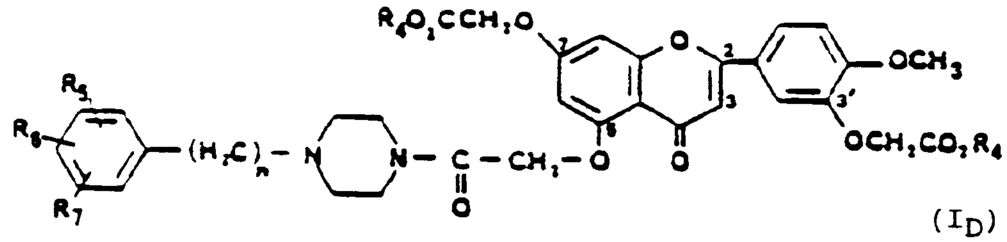

($I_D$)

in which the meanings of n, $R_4$, $R_5$, $R_6$ and $R_7$ are identical to those given for formula I,

- or

with compounds of formula $III_B$, compounds of the general formula $I_E$

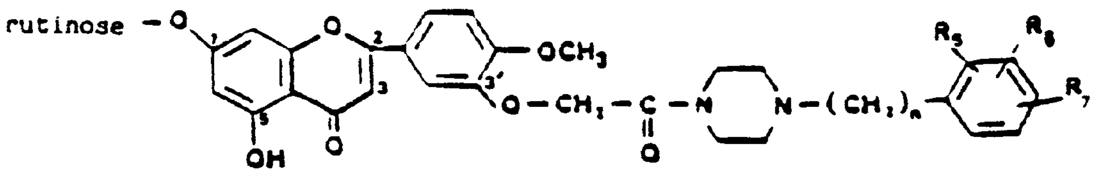

($I_E$)

in which n, $R_5$, $R_5$ and $R_7$ are as defined for formula I,

which are then subjected to enzymatic hydrolysis using naringinase to yield compounds of the general formula $I_F$ :

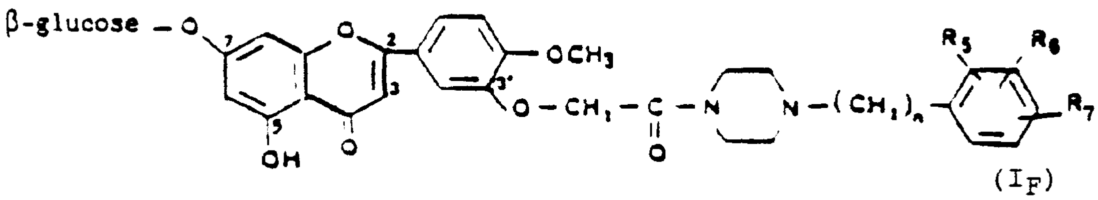

($I_F$)

in which n, $R_5$, $R_5$ and $R_7$ are as defined above,

which are then

subjected to enzymatic hydrolysis using β-glucosidase to yield compounds of the general formula $I_A$,

which are then condensed with a compound of the general formula III to yield compounds of the general formula $I_B$,

or :

a compound of the general formula XI :

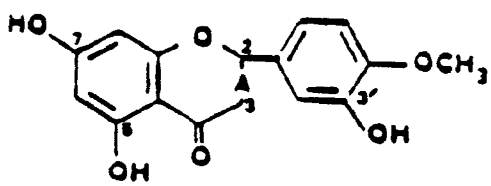

(XI)

in which A is as defined for formula I, is reacted with an appropriate amount of compounds of the general formula $III_B$,

to yield compounds of the general formula $I_G$ :

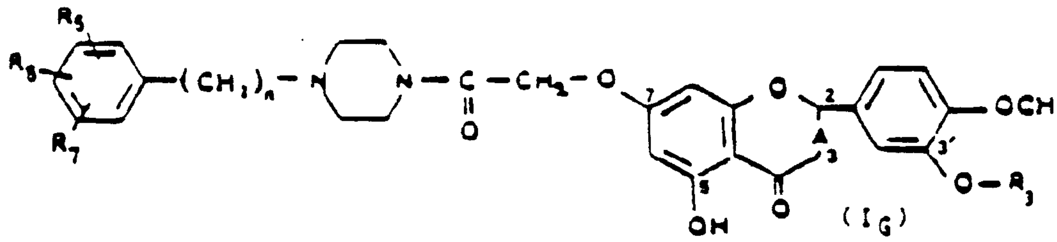

$(I_G)$

in which n, A, $R_5$, $R_5$ and $R_7$ are as defined for formula I, and $R_3$ represents a hydrogen atom when the quantity of compounds of the general formula $III_B$ used is approximately equimolar, or a radical of the formula W when the alkylation is carried out with at least double the amount of alkyl chloride of the general formula $III_B$,

and, if desired, the compounds of formulae $I_A$ to $I_G$, which form the totality of the compounds of formula I, are then converted into salts with a pharmaceutically acceptable mineral or organic acid.

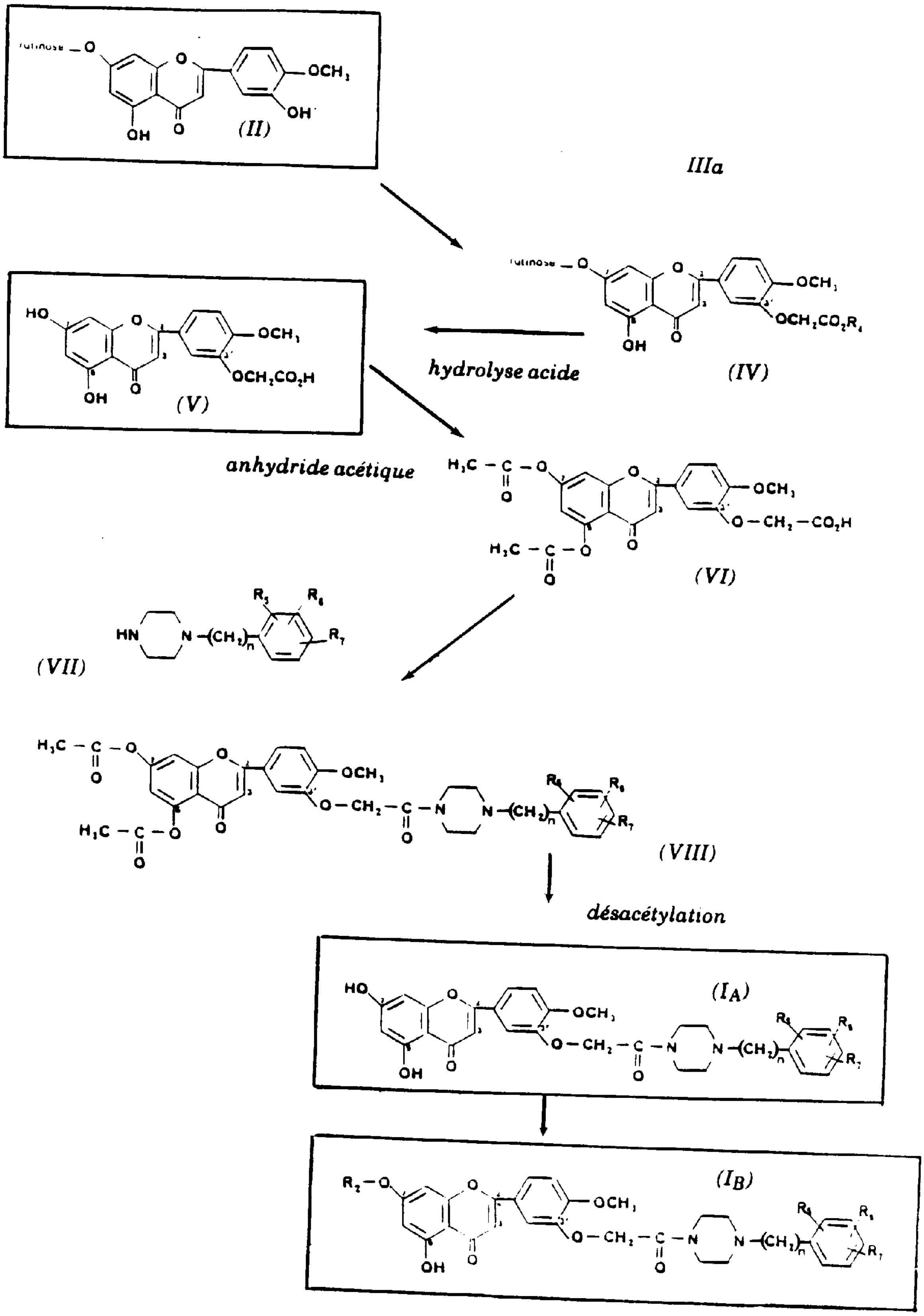
(II)
IIIa
(IV)
hydrolyse acide
(V)
anhydride acétique
(VI)
(VII)
(VIII)
désacétylation
($I_A$)
($I_B$)

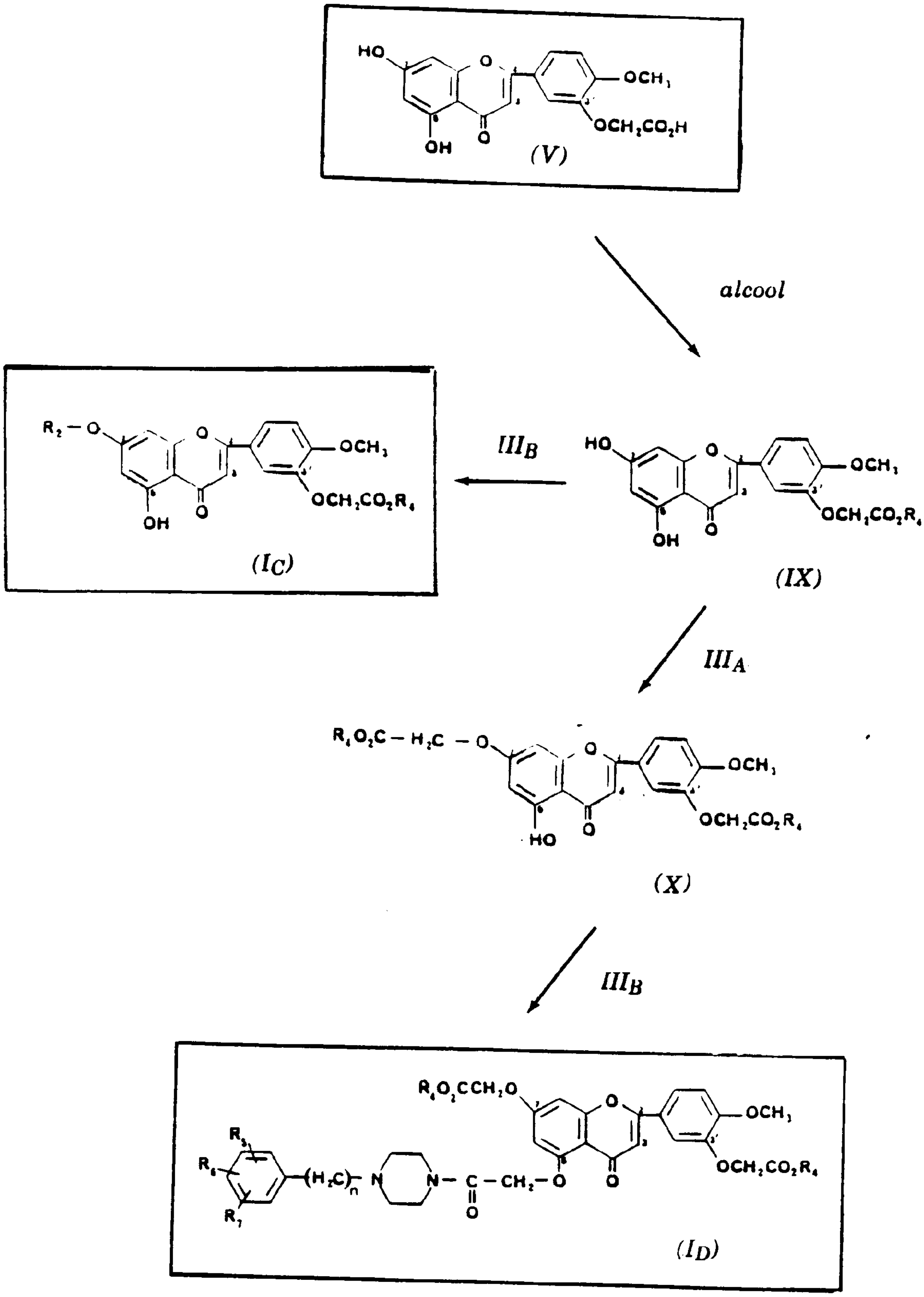


(V)

$(I_C)$

(IX)

(X)

$(I_D)$

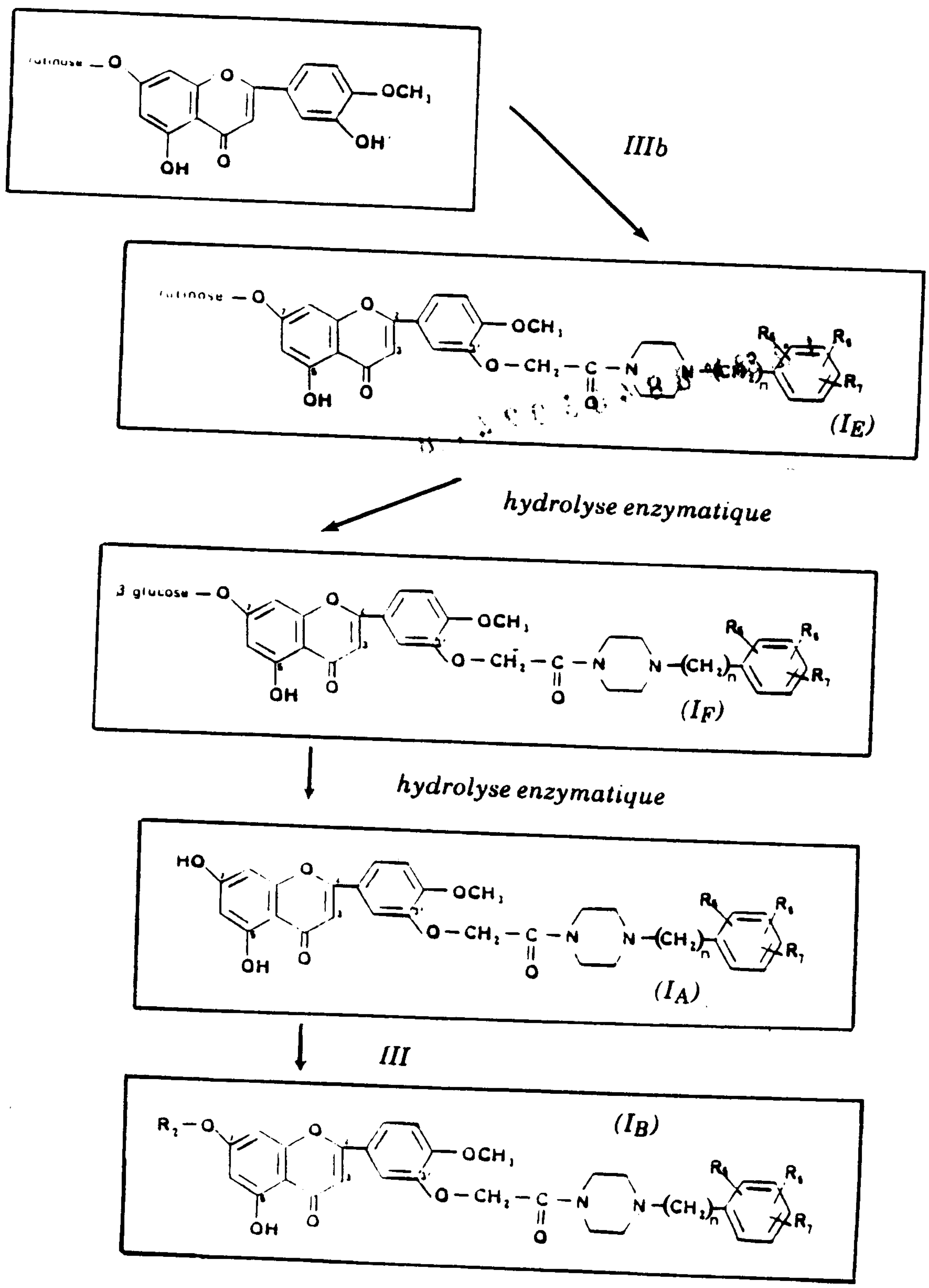
IIIb
(IE)
hydrolyse enzymatique
(IF)
hydrolyse enzymatique
(IA)
III
(IB)